# EUROPÄISCHE PATENTSCHRIFT

(11) **EP 4 076 294 B1**
(45) Veröffentlichungstag und Bekanntmachung des Hinweises auf die Patenterteilung: **16.07.2025**
(21) Anmeldenummer: 20839264.7
(22) Anmeldetag: 17.12.2020
(51) Int. Cl.: A61F 2/91, A61B 17/12, A61F 2/82, A61F 2/06

(54) **MEDIZINISCHES SET, MEDIZINISCHES SYSTEM UND ABDECKVORRICHTUNG ZUR BEHANDLUNG VON ANEURYSMEN**
MEDICAL KIT, MEDICAL SYSTEM, AND COVERING DEVICE FOR THE TREATMENT OF ANEURYSMS
ENSEMBLE MÉDICAL, SYSTÈME MÉDICAL ET DISPOSITIF DE RECOUVREMENT DESTINÉ AU TRAITEMENT DE L'ANÉVRISME

(30) Priorität: 20.12.2019 DE 102019135502
(43) Veröffentlichungstag der Anmeldung: 26.10.2022
(73) Patentinhaber: Acandis GmbH, 75177 Pforzheim (DE)
(72) Erfinder: BÜCHERT, Michael, 75177 Pforzheim (DE); CATTANEO, Giorgio, 76199 Karlsruhe (DE)
(74) Vertreter: Meissner Bolte Partnerschaft mbB
(86) Internationale Anmeldenummer: PCT/EP2020/086616
(87) Internationale Veröffentlichungsnummer: WO 2021/122877

(56) Entgegenhaltungen:
- WO-A1-2019/175341
- US-A1- 2004 054 406
- US-A1- 2014 058 498
- US-A1- 2019 298 390

## Beschreibung

Die Erfindung betrifft ein medizinisches Set zur Behandlung von Gefäßmalformationen, insbesondere Aneurysmen und/oder Fisteln. Ferner betrifft die Erfindung ein medizinisches System sowie eine Abdeckvorrichtung zur Behandlung von Gefäßmalformationen.

WO 2014/177634 A1 beschreibt einen hochflexiblen Stent, der eine komprimierbare und expandierbare Gitterstruktur aufweist, wobei die Gitterstruktur einstückig ausgebildet ist. Die Gitterstruktur umfasst geschlossene Zellen, die durch jeweils vier Gitterelemente begrenzt sind. Die Gitterstruktur weist wenigstens einen Zellenring auf, der zwischen drei und sechs Zellen umfasst.

Aus der Praxis der Anmelderin sind außerdem Stents mit Gitterstrukturen bekannt, die aus einem einzigen Draht gebildet sind. Der Draht ist mit sich selbst verflochten, um ein röhrchenförmiges Geflecht zu bilden. An den axialen Enden des röhrchenförmigen Geflechts ist der Draht umgelenkt, so dass sich atraumatisch wirkende Schlaufen bilden. Die axialen Enden können trichterförmig ausgeweitet sein.

Die bekannte medizinische Vorrichtung eignet sich insbesondere zur Behandlung von Aneurysmen in kleinen, zerebralen Blutgefäßen. Derartige Blutgefäße weisen einen sehr kleinen Querschnittsdurchmesser auf und sind oft stark gewunden. Der bekannte Stent ist dazu hochflexibel gestaltet, so dass er einerseits auf einen sehr kleinen Querschnittsdurchmesser komprimierbar ist und andererseits eine hohe Biegeflexibilität aufweist, die die Zuführung in kleine zerebrale Blutgefäße ermöglicht.

Zur Behandlung von Aneurysmen in zerebralen Blutgefäßen ist es zweckmäßig, Stents einzusetzen, die sich über ein Aneurysma spannen und dieses vom Blutfluss innerhalb des Blutgefäßes abschirmen. Um dies zu ermöglichen, ist es bekannt, Stents mit einer Abdeckung zu versehen, die die Zellen des Stents verschließt und so einen Blutstrom in ein Aneurysma verhindert.

Eine weitere ergänzende oder alternative Behandlungsmethode von Aneurysmen ist die Implantation sogenannter Coils in das Aneurysma, welche dort zu einer Blutgerinnung führen. Der resultierende Thrombus verhindert dann die Blutzirkulation im Aneurysma und somit das Risiko einer Ruptur mit anschließender Blutung.

Besonders bei breithalsigen Aneurysmen neigen die Coils jedoch dazu, während der Implantation in die Blutbahn zu wandern und somit einen Verschluss des Hauptgefäßlumen zu verursachen. Bei der Technik "Ballon assisted Coiling" werden Katheter mit "Compliance-Ballonen" im Gefäß, speziell an der Höhe des Aneurysmahalses, positioniert. Der mit Kontrastmittel gefüllte Ballon schließt den Aneurysmahals während der Platzierung der Coils und zwingt die Coils in eine kompakte Anordnung innerhalb des Aneurysma-Raums. Weil Coils plastisch verformbar sind, bleiben diese dann in ihrer Form. Sie verlassen das Aneurysma auch dann nicht, wenn der Ballon entfernt wird. Ein Problem besteht jedoch hierbei insbesondere aufgrund der Tatsache, dass der Ballon das Gefäß verschließt. Bei anhaltender Prozedur (bei großen Aneurysmen werden mehrere Coils platziert, die Prozedur kann mehrere Minuten dauern), wird die Blutströmung in der Zeit vollständig unterbrochen. Zwar sorgen Kollateralgefäße für eine Versorgung von nachgelagertem Gewebe, trotzdem bleibt die Gefahr einer Unterperfusion bestehen.

Weiterhin wird während der Prozedur ein Katheter, durch den die Coils geführt werden, an der Seite des Ballons "gejailt", das heißt festgeklemmt. Bei der evtl. Notwendigkeit, Coil-Katheter zu wechseln (z.B im Fall einer Beschädigung) bei noch nicht vollständiger Prozedur, soll der Ballon deflatiert (entleert) werden, um den Katheter zurückziehen zu können. In dieser Phase kann es zu einer Verschiebung der Coils, die noch nicht vollständig und kompakt in dem Gefäß liegen, kommen. Diese können dann zu einem Gefäßverschluss führen.

Zwar ist aus WO 2019/175 341 A1 ein Stent bekannt, der eine elektrogesponnene Abdeckung aufweist und dazu vorgesehen ist, ein Aneurysma abzudecken. Einen ähnlichen Stent beschreibt US 2014/058 498 A1. Solche Stents können jedoch nicht genutzt werden, um Embolisationsmitteln in dem Aneurysma zurückzuhalten. Zum Einbringen der Embolisationsmittel muss die Abdeckung von einem Mikrokatheter durchdrungen werden, wobei die Gefahr besteht, dass nach Entfernen des Mikrokatheters eine Öffnung verbleibt, über welche die Embolisationsmitteln in das freie Blutgefäß treten können.

Vor diesem Hintergrund ist es Aufgabe der Erfindung, ein medizinisches Set zur Behandlung von Gefäßmalformationen anzugeben, mit dessen Hilfe die Gefahr von Gefäßverschlüssen zumindest reduziert ist. Eine weitere Aufgabe der Erfindung besteht darin, ein medizinisches System anzugeben.

Erfindungsgemäß wird diese Aufgabe im Hinblick auf das medizinische Set durch den Gegenstand des Patentanspruchs 1, im Hinblick auf das medizinische System durch den Gegenstand des Patentanspruchs 14 und im Hinblick auf die Abdeckvorrichtung durch den Gegenstand des Patentanspruchs 15 gelöst.

Konkret wird die Aufgabe gelöst durch ein medizinisches Set zur Behandlung von Gefäßmalformationen, insbesondere Aneurysmen und/oder Fisteln, z.B. Carotid Direct Cavernous Fistula gelöst. Das Set weist eine permanent implantierbare Abdeckvorrichtung, insbesondere einen Stent, zum Abdecken der Gefäßmalformation auf. Die Abdeckvorrichtung weist eine röhrchenförmige, selbstexpandierbare Gitterstruktur und eine Abdeckung aus einem elektrogesponnenen Gewebe auf. Die Abdeckung ist mit der Gitterstruktur verbunden und überdeckt zumindest teilweise die Gitterstruktur, um im implantierten Zustand über der Gefäßmalformation platziert zu werden. Das medizinische Set weist ein Embolisationsmittel auf, das durch ein Zuführmittel im implantierten Zustand zur Behandlung der Gefäßmalformation applizierbar ist. Die Abdeckung bildet eine poröse Membran, die von dem Zuführmittel zur Applikation des Embolisationsmittels durchdringbar und dazu angepasst ist, sich im durchdrungenen Zustand an den Außenumfang des Zuführmittels anzulegen.

Das erfindungsgemäße medizinische Set umfasst also die Abdeckvorrichtung und das Embolisationsmittel. Das Set kann weitere Komponenten umfassen.

Im Unterschied zu dem medizinischen Set gemäß DE 10 2019 121 546 vom 09. August 2019 ist das erfindungsgemäße medizinische Set dazu vorgesehen, dauerhaft und nicht nur temporär ein Aneurysma abzudecken. Die Abdeckvorrichtung bildet ein permanentes Implantat, wie beispielsweise einen Stent. Die permanent implantierbaren Abdeckvorrichtung wird dazu mithilfe eines Transportdrahts durch das Zuführmittel in bekannter Weise über dem zur behandelnden Aneurysma platziert. Nachdem die Abdeckvorrichtung vollständig aus dem Zuführmittel entlassen wurde, wird diese vom Transportdraht gelöst. Die Abdeckvorrichtung ist damit fest und permanent im Gefäß verankert und kann im final implantierten Zustand nicht mehr in das Zuführmittel eingezogen werden.

Die Abdeckvorrichtung ist dazu beim Einführen lösbar mit einem Transportdraht verbunden.

Die Abdeckvorrichtung ist vorzugsweise ein Stent. Generell weist die Abdeckvorrichtung eine röhrchenförmige und selbstexpandierbare Gitterstruktur auf. Die Gitterstruktur ist am distalen und proximalen Ende, also an beiden axialen Enden offen, sodass diese im expandierten Zustand in bekannter Weise von Blut durchströmt werden kann. Im Unterschied dazu weisen bekannte Thrombektomie-Devices zum Wiedereinziehen in einen Katheter einen nach innen sich schließenden trichterförmigen Abschnitt auf, der fest, also nicht lösbar, mit dem Transportdraht verbunden ist.

Die aus dem elektrogesponnenen Gewebe gebildete Abdeckung auf der Gitterstruktur weist eine solche Ausdehnung auf, dass eine Gefäßmalformation, insbesondere ein Aneurysma, im implantierten Zustand ausreichend sicher abgedeckt werden kann. Die Abdeckung kann sich dabei über den gesamten Umfang der Gitterstruktur und in ausreichend großer Länge in axialer Richtung der Gitterstruktur erstrecken. Es ist auch möglich, dass sich die Abdeckung nicht über den gesamten Umfang, sondern nur über einen Teilumfang der Gitterstruktur, insbesondere über ein Winkelsegment der Gitterstruktur, und/oder eine Teillänge erstreckt.

Die Abdeckung ist mit der Gitterstruktur so verbunden, dass diese sich im implantierten Zustand nicht von der Gitterstruktur lösen kann. Das elektrogesponnene Gewebe der Abdeckung bespannt die Gitterstruktur.

Generell handelt es sich um Embolisationsmittel, die zur Behandlung von Gefäßmalformationen geeignet sind. Bei den Embolisationsmitteln, die zum Veröden in das Aneurysma eingebracht werden können, handelt es sich beispielsweise, aber nicht ausschließlich um ein Coil oder mehrere Coils und/oder um embolische Flüssigkeiten, (liquid embolics), wie viskose Hydrogele.

Erfindungsgemäß bildet die Abdeckung, konkret das elektrogesponnene Gewebe der Abdeckung eine poröse Membran. Dabei kann die gesamte Abdeckung, konkret das elektrogesponnene Gewebe der Abdeckung, die poröse Membran bilden. Es ist auch möglich, dass nur ein Teil der Abdeckung die poröse Membran bildet. Die poröse Membran ist so ausgebildet, dass diese von dem Zuführmittel zur Applikation des Embolisationsmittels durchdringbar ist. Mit anderen Worten ist die poröse Membran so ausgebildet, dass das Zuführmittel vom Innenlumen der implantierten Gitterstruktur aus radial bzw. in einem spitzen Winkel nach außen durch die Membran hindurch sondiert werden kann. Das Zuführmittel durchstößt bzw. durchdringt dabei die Membran, bzw. das elektrogesponnene Gewebe der Abdeckung, ohne die Abdeckung bzw. das Gewebe irreversibel zu verletzen. Dabei ist es möglich, dass einzelne Poren des Gewebes sich stark aufweiten oder vergrößern.

Es hat sich gezeigt, dass das elektrogesponnene Gewebe ausreichend flexibel ist, sodass die im elektrogesponnenen Gewebe vorhandenen Poren oder eine Pore durch das Zuführmittel elastisch aufgeweitet werden können, wenn das Zuführmittel das elektrogesponnene Gewebe durchdringt. Dabei verformen sich die Poren bzw. verformt sich die Pore und das um die Poren/Pore herum angeordnete Material der Membran so elastisch, dass eine ausreichend große Öffnung für das Zuführmittel entsteht. Das Zuführmittel selbst weist einen Durchmesser auf, der Größenordnungen größer ist als der Durchmesser der Poren. Das elektrogesponnene Gewebe bzw. die poröse Membran ist so elastisch, dass eine ausreichende Verformung der Poren möglich ist, um das Zuführmittel durchzulassen.

Einer beispielhaften, vorgenannten Größenordnung des Durchmessers des Zuführmittels werden bspw. Porengrößen (Inkreis-Durchmesser) der Abdeckung bzw. der Bespannung von 5-20µm bei einer 1-Minute besponnenen Gitterstruktur oder Porengrößen (Inkreis-Durchmesser) von 1-5µm bei einer 2-Minuten besponnenen Gitterstruktur zu Grunde gelegt. Wo in der Beschreibung die Einheit "Zoll" benutzt wird, gilt die folgende Umrechnung:
1" = 25,4mm

Der Außendurchmesser des geeigneten Zuführmittels, bspw. ein Führungsdraht (Guidewire) mit nachfolgendem Mikrokatheter kann dazu wie folgt beschrieben werden:
Außendurchmesser des Führungsdrahtes: ca. 0,36 mm (0.014")
Außendurchmesser des Mikrokatheters: ca. 2.1 Fr (0,70mm)
Innendurchmesser des Mikrokatheters (ID): ca. 0,42mm (0.0165")

Kleinere Kombinationen aus Außendurchmesser des Führungsdrahtes von ca. 0,254mm (0.010") und Außendurchmesser des Mikrokatheters von ca. 1.5 Fr (0,5 mm), ID: 0,33mm (0.013") sind möglich.

Die vorstehend genannten Größenverhältnisse sind beispielhaft. Andere Größenverhältnisse von Poren und Zuführmittel sind möglich.

In diesem Zustand, also wenn das Zuführmittel die Membran durchdringt, legt sich die poröse Membran der Abdeckung bzw. des elektrogesponnenen Gewebes an den Außenumfang des Zuführmittels an und dichtet dieses so gegen die Gefäßmalformation, insbesondere das Aneurysma ab. Das Embolisationsmittel kann dann durch das Zuführmittel durch die Membran hindurch in das Aneurysma transportiert werden. Das Zuführmittel ist dabei im Lumen der Abdeckvorrichtung bzw. des Stents angeordnet. Durch die Membranfunktion der Abdeckung bzw. des Gewebes ist es möglich, dass das Embolisationsmittel mithilfe des Zuführmittels durch das Lumen der Abdeckvorrichtung radial bzw. in einem spitzen Winkel von innen nach außen durch die Wandung bzw. die Gitterstruktur der Abdeckvorrichtung in das Aneurysma eingebracht werden kann.

Die Abdichtung des Zuführmittels durch das Gewebe bzw. die Abdeckung verhindert, dass das Embolisationsmittel beim Einbringen in das Aneurysma in die Blutbahn gelangen kann.

Nachdem das Aneurysma ausreichend mit dem Embolisationsmittel gefüllt ist, kann das Zuführmittel durch das Lumen der Gitterstruktur entfernt werden. Dabei hilft wiederum die Flexibilität der Abdeckung bzw. des elektrogesponnenen Gewebes, das ein Entfernen des Zuführmittels ermöglicht, ohne dass dabei das Embolisationsmittel aus dem Aneurysma austreten kann.

Die Membranfunktion der Abdeckung bzw. des Gewebes kann wie folgt beschrieben werden. Die Abdeckung bzw. das Gewebe ist einerseits derart geringfügig porös, dass das Embolisationsmittel im implantierten Zustand der Abdeckvorrichtung durch die Abdeckung im Aneurysma zurückgehalten wird. Andererseits ist die Abdeckung bzw. das Gewebe so offenporig und flexibel, dass ein Zuführmittel, beispielsweise ein Mikrokatheter, durch die Abdeckung bzw. durch das Gewebe hindurch geführt werden kann, ohne dass die Abdeckung bzw. das Gewebe dabei zerstört oder von der Stent-Trägerstruktur bzw. allgemein von der Gitterstruktur abgelöst werden.

Bei einem elektrogesponnenen Gewebe sind Poren üblicherweise unregelmäßig gestaltet. Das Herstellungsverfahren erlaubt es jedenfalls nicht, eine musterartige Anordnung oder Gestaltung von Poren zu erstellen. Allerdings können die Porengrößen anhand der Prozessparameter zumindest soweit eingestellt werden, dass sichergestellt ist, dass wenigstens ein Teil der Poren eine gewisse Mindestgröße aufweist.

Beispielsweise kann der Prozess des Elektrospinnens unmittelbar auf der Gitterstruktur erfolgen, so dass bei der Bildung der Abdeckung gleichzeitig eine Verbindung mit der Gitterstruktur hergestellt wird. Die Abdeckung kann mit der Gitterstruktur stoffschlüssig verbunden sein. Beispielsweise kann die Abdeckung mit der Gitterstruktur durch eine Klebeverbindung verbunden sein. Die Klebeverbindung kann durch einen Haftvermittler hergestellt werden. Der Haftvermittler kann beispielsweise Polyurethan umfassen oder daraus bestehen.

Die Abdeckung aus einem elektrogesponnenen Gewebe ist darüber hinaus äußerst dünn und flexibel, was die Flexibilität der Gitterstruktur nur unwesentlich bis nicht beeinträchtigt. Insbesondere hindert die Abdeckung die Gitterstruktur im Unterschied zu vorbekannten Abdeckungen, die aus Textilmaterialien hergestellt sind, kaum an der Komprimierung. Insgesamt lässt sich also die gesamte Abdeckvorrichtung auf einen erheblich kleineren Querschnittsdurchmesser komprimieren und so über kleine Katheter in besonders kleine Blutgefäße führen. Dies ist insbesondere für die Behandlung von Aneurysmen in zerebralen Blutgefäßen relevant, wozu sich die Erfindung besonders eignet.

Mit dem erfindungsgemäßen medizinischen Set sind daher auch Behandlungen in Blutgefäßen möglich, die mit bisherigen medizinischen Vorrichtungen, die eine Gitterstruktur und eine Abdeckung aufweisen, nicht erreicht werden können. Wegen der hohen Komprimierbarkeit der Abdeckvorrichtung treten bei der Zuführung durch einen Katheter bzw. allgemein durch ein Zuführmittel sehr geringe Zuführkräfte auf. Insbesondere können die Zuführkräfte bei der Abdeckvorrichtung im Vergleich zur Zuführung einer Gitterstruktur ohne Abdeckung sogar gleich sein.

Weiterhin kann durch die Abdeckvorrichtung, welche zweckdienlicherweise am Behandlungsort, also auf Höhe des Aneurysmas angeordnet ist, beim und nach dem Setzen der Coils ein Herauswandern der Coils aus dem Aneurysma verhindert werden, sodass die Gefahr eines durch die Coils verursachten Gefäßverschlusses ebenfalls zumindest stark reduziert und vorzugsweise ausgeschlossen werden kann.

Die Abdeckung ist porös und insbesondere blutdurchlässig. Unter porös kann hierbei verstanden werden, dass die Abdeckung bzw. das elektrogesponnene Gewebe netzartig oder als ein Netz ausgebildet ist. Dem liegt der Gedanke zugrunde, dass im Bereich der Abdeckung befindliche Zellen mit Blut und somit mit Nährstoffen versorgt werden können, sodass hierdurch keine Unterversorgung beim Setzen der Coils oder danach erfolgt.

Das erfindungsgemäße medizinische Set ermöglicht somit eine gute Abschirmung eines Aneurysmas zum Zurückhalten des in das Aneurysma eingesetzten Embolisationsmittels, bspw. Coils, lässt jedoch gleichzeitig eine Nährstoffzufuhr in das Aneurysma zu. Auch die Nährstoffzufuhr in abzweigende Blutgefäße und angrenzende Gefäßinnenwände wird durch das medizinische Set erreicht, die von der Abdeckung überdeckt werden. Die Abdeckung, die aus dem elektrogesponnenen Gewebe gebildet ist, ermöglicht eine Abdeckung eines Aneurysmas, lässt jedoch zeitgleich eine gewisse Durchlässigkeit für Blut zu. Diese Durchlässigkeit ist zweckmäßig, um die Zellen der Aneurysmenwand mit Nährstoffen zu versorgen. Dadurch wird eine Degeneration der Zellen und eine daraus möglicherweise resultierende Ruptur des Aneurysmas vermieden.

Die Vorteile der Erfindung kommen nicht nur bei der Behandlung von Aneurysmen zum Tragen, sondern auch bei anderen Gefäßmalformationen wie z.B. bei Fisteln, z.B. Carotid Direct Cavernous Fistula.

Die Erfindung hat verschiedene weitere Vorteile.

Das Einbringen von Coils oder embolischen Flüssigkeiten lässt sich bei konventionellen Behandlungsmethoden mit z.B. Flow-Divertern nur im Voraus planen. Hierzu wird üblicherweise ein zweiter Katheter zuerst in das Aneurysma eingeführt, bevor der Stent oder Flow-Diverter vor dem Aneurysma-Hals und über dem zweiten Katheter platziert wird. Der zweite Katheter befindet sich radial außerhalb des Stents bzw. des Flow-Diverter zwischen der Katheterwand und der Gefäßwand. Durch den zweiten Katheter können dann Coils oder Flüssig-Embolisate in das Aneurysma geleitet werden. Wenn das Aneurysma ausreichend befüllt ist wird der zweite Katheter entfernt.

Ein späteres Coilen oder Zuführen von Flüssig-Embolisaten ist nicht mehr möglich, da man ohne den zweiten Katheter nicht mehr in das Aneurysma gelangt.

Demgegenüber bietet die Erfindung den Vorteil, dass das elektrogesponnene Gewebe eine Membran-Funktion übernimmt. Dies ermöglicht erweiterten Handlungsspielraum für den Anwender direkt bei der Applikation oder selbst Monate oder Jahre später:
Die mit dem elektrogesponnenen Gewebe bespannte Abdeckvorrichtung, insbesondere der bespannte Stent, wird zunächst über den Aneurysma-Hals gelegt. Im Anschluss (oder bei Bedarf zu einem späteren Zeitpunkt) kann das Zuführmittel, insbesondere ein Mikrokatheter, welcher bereits für die Zuführung des Stents genutzt wurde, für die Passage durch die bespannte Gewebeschicht genutzt werden. Hierzu wird bspw. ein Führungssdraht, vorzugsweise in der Ausführung 0.014" oder 0.012" oder 0.010" steif, in den Mikrokatheter eingelegt und vorher nach Belieben an der Spitze zurecht geformt (z.B. 90° Biegung, enger Radius). Nun kann eine beliebige Stentzelle, bzw. allgemein eine Zelle der Gitterstruktur, welche den Aneurysma-Hals abdeckt, mit dem Führungsdraht sondiert werden. Wurde die Zelle erfolgreich sondiert, kann der Mikrokatheter auf dem Führungsdraht hinterher durch die Zelle in das Aneurysma geschoben werden. Anschließend wird der Führungsdraht entfernt, sodass Coils oder Flüssig-Embolisate durch den Katheter zugeführt werden können.

Das Gewebe reagiert hierbei wie eine Membran, die sich eng um den Führungsdraht bzw. Mikrokatheter legt und gleichzeitig verhindert, dass Embolisationsmittel, beispielsweise Coils oder Embolisat in die Blutbahn austreten/austritt.

Das Einbringen des Embolisationsmittels in das Aneurysma mit dem erfindungsgemäßen Set erfolgt zeitlich nach dem Implantieren der Abdeckvorrichtung. Der zeitliche Abstand ist variabel und kann sogar Monate oder Jahre betragen. Ein zweiter Katheter, der vor dem Implantieren der Abdeckvorrichtung gelegt wird, entfällt. Optional kann der Mikrokatheter direkt nach Absetzen des Stents verwendet werden, um die Membran zu sondieren und das Embolisat bzw. allgemein das Embolisationsmittel zuzuführen. Die Erfindung erleichtert so das Einbringen des Embolisationsmittels in das Aneurysma beträchtlich, weil das Implantieren der Abdeckvorrichtung und das Einbringen des Embolisationsmittels in das Aneurysma durch ein und dasselbe Zuführmittel erfolgen kann.

Nach dem nebengeordneten Systemanspruch 14 wird die Kombination des erfindungsgemäßen Sets mit dem Zuführsystem offenbart und beansprucht.

Die erfindungsgemäße Abdeckvorrichtung gemäß nebengeordnetem Anspruch 15 wird unabhängig von dem Embolisationsmittel und dem Zuführmittel offenbart und beansprucht. Die erfindungsgemäße Abdeckvorrichtung gemäß nebengeordnetem Anspruch 15 ist also weder auf das Embolisationsmittel noch auf das Zuführmittel eingeschränkt.

Bevorzugte Ausgestaltungen, Weiterbildungen und Varianten sind Gegenstand der Unteransprüche.

So kann die poröse Membran der Abdeckung dazu angepasst sein, eine durch das Zuführmittel beim Durchdringen der Membran gebildete Öffnung nach dem Entfernen des Zuführmittels zumindest teilweise zurückzubilden.

Nachdem das Aneurysma ausreichend mit Coils/Embolisat befüllt wurde, kann das Zuführmittel, insbesondere der Mikrokatheter, sicher aus dem Aneurysma gezogen werden. Die Eigenschaften der Membran führen zu einem sofortigen Verschluss der Öffnung der Zelle der Gitterstruktur, insbesondere der Stent-Zelle, um ein Austreten von Coils bzw. Embolisat in das Hirngefäß zu verhindern. Die Zelle der Gitterstruktur muss dabei nicht vollständig verschlossen sein, um diese Wirkung hervorzubringen.

Mit anderen Worten schließt sich, sobald das Zuführmittel vollständig aus dem Aneurysma entfernt ist und damit aus der Abdeckung bzw. dem Gewebe herausgezogen ist, die Abdeckung bzw. das Gewebe zumindest teilweise wieder. Die für das Zuführmittel aufgeweitete, flexible Öffnung in der Abdeckung bzw. im Gewebe bildet sich auf Grund der Elastizität des Gewebes zumindest teilweise zurück, sodass das Gewebe eine im Wesentlichen geschlossene, wenn auch poröse Abdeckung bildet, die das im Aneurysma angeordnete Embolisationsmittel sicher zurückhält. Die Abdeckung bildet damit eine poröse Membran, die zum Einbringen des Embolisationsmittels in das Aneurysma von dem Zuführmittel durchstoßen bzw. durchdrungen werden kann und dieses beim Einbringen abgedichtet. Nach dem Entfernen des Zuführmittels verschließt sich die Membran wieder, um so das Embolisationsmittel im Aneurysma zurückzuhalten. Der Verschluss muss nicht vollständig sein, um das Embolisationsmittel zurückhalten zu können. Bei einer weiteren bevorzugten Ausführungsform ist vorgesehen, dass die poröse Membran der Abdeckung dazu angepasst ist, die Öffnung auf höchstens 80%, insbesondere höchstens 60%, insbesondere höchstens 40%, insbesondere höchstens 20% des Durchmessers des Zufuhrmittels zurückzubilden.

Die zurückgebildete Öffnung ist kleiner, als der Außendurchmesser des Zuführmittels, mit dem das Zuführmittel die Membran durchdrungen hat.

Vorzugsweise ist die Abdeckung derart geringfügig porös, dass diese im implantierten Zustand das applizierte Embolisationsmittel zurückhält.

Die Gitterstruktur weist zweckdienlicherweise eine Formgedächtnislegierung, insbesondere Nitinol oder eine andere Formgedächtnislegierung, auf oder ist bevorzugt aus einem derartigen Material gebildet. Die Gitterstruktur kann geflochten oder durch Laserschneiden hergestellt sein. Für zerebrale Anwendungen wird eine geflochtene Gitterstruktur bevorzugt.

Um eine ausreichende Flexibilität der Abdeckung zu erreichen, ist diese vorzugsweise aus unregelmäßig netzartig angeordneten Fäden gebildet, die eine Fadendicke zwischen 0,1 µm und 3 µm, insbesondere zwischen 0,2 µm und 2 µm, insbesondere zwischen 0,5 µm und 1,5 µm, insbesondere zwischen 0,8 µm und 1,2 µm, aufweisen.

Die Fäden können vorzugsweise eine Fadendicke von höchstens 2 µm, insbesondere höchstens 1,5 µm, insbesondere höchstens 1 µm, und eine Fadendicke von wenigstens 0,3 µm aufweisen

Zweckdienlicherweise weist die Abdeckung eine Porosität von höchstens 70%, insbesondere von höchstens 50%, insbesondere höchstens 40%, insbesondere höchstens 30% auf. Hierdurch wird die Stabilität der Abdeckung zum einen in Bezug auf eine Kraft der Coils bzw- allgemein des Embolisationsmittels erhöht, welche beispielsweise beim im Aneurysma eingesetzten Zustand, auf die Abdeckung drückt. Zum anderen wird die Stabilität der Abdeckung im Hinblick auf eine Bruchstabilität vorteilhaft optimiert.

In einer Ausführungsform weist die Abdeckung eine Porosität von wenigstens 5%, insbesondere von wenigstens 10%, insbesondere von wenigstens 20%, insbesondere von wenigstens 30%, insbesondere von wenigstens 40% und insbesondere von wenigstens 45% auf. Dieser Ausführungsform liegt der Gedanke zugrunde, dass durch eine bestehende Porosität in Höhe der zuvor genannten Prozentangaben - wie bereits vorstehend erwähnt - eine Versorgung von beispielsweise Seitengefäßen oder Gefäßwänden in der Nähe des Aneurysmas während der Platzierung des Embolisationsmittels und danach gewährleistet ist.

Außerdem ist die Abdeckung aufgrund ihrer Porosität speziell für Mikrokatheter geeignet, da die Abdeckung komprimierbar ist und somit mit niedrigeren Reibungskräften durch die Mikrokatheter an den Behandlungsort einführbar und auch zurückführbar ist. Unter Mikrokatheter kann hierbei ein Katheter verstanden werden, der einen Innendurchmesser mit einem Wert im Bereich von 0,3mm bis 0,75mm aufweist.

Die vorstehenden Ober- und Untergrenzen können zu Bereichen kombiniert werden (s.a. Ansprüche 5, 6), soweit sinnvoll.

Gemäß einer zweckdienlichen Ausgestaltung erstreckt sich die Abdeckung über die gesamte Umfangsfläche der Gitterstruktur. Hierdurch werden die zuvor genannten Vorteile, insbesondere hinsichtlich einer Stabilität der Abdeckung und somit der Gitterstruktur vorteilhaft optimiert.

Gemäß einer zweckdienlichen Weiterbildung erstreckt sich die Abdeckung über einen Teil, insbesondere höchstens 70%, insbesondere höchstens 60%, insbesondere höchstens 50%, insbesondere höchstens 40%, insbesondere höchstens 30% des Umfangs der Gitterstruktur. Bevorzugt erstreckt sich somit die Abdeckung somit lediglich über den Behandlungsort, also beispielsweise über eine Öffnung des Aneurysmas. Durch diese Weiterbildung wird sichergestellt, dass zum einen eine Öffnung des Aneurysmas zum Fixieren der darin platzierten Coils ausreichend verschlossen ist. Zum anderen ist hierdurch sichergestellt, dass insbesondere sich auf Höhe des Aneurysmas befindliche Zellen und/oder Seitengefäße durch die fehlende Abdeckung besser und weiterhin mit Blut und somit mit Nährstoffen versorgt werden können.

Gemäß einer ergänzenden oder alternativen Ausführungsform erstreckt sich die Abdeckung bei mindestens 80 %, insbesondere über mindestens 90 % und insbesondere über 100 % der Länge L der Gitterstruktur. Die Länge L entspricht der Gesamtlänge der Abdeckvorrichtung, insbesondere des Implantates.

Diese Ausführungsform ist insbesondere geeignet für fusiforme bzw. langhalsige Aneurysmen, in denen ein längerer Abschnitt abgedeckt werden soll. Unter fusiformen Aneurysmen werden hierbei Aneurysmen verstanden, die sich über wenigstens 50%, insbesondere über wenigstens 75% des gesamten Umfangs oder über den gesamten Umfang eines Blutgefäßes erstrecken.

In einer Ausführungsform erstreckt sich die Abdeckung über höchstens 80%, insbesondere über höchstens 60%, insbesondere über höchstens 40% der Länge der Gitterstruktur, wobei die Abdeckung vom distalen und/oder vom proximalen Ende der Gitterstruktur beabstandet ist. Die Länge L entspricht der Gesamtlänge der Abdeckvorrichtung, insbesondere des Implantates. Durch die hiermit nicht vollständige Abdeckung des zylindrischen Bereichs der Gitterstruktur wird erreicht, dass eventuelle Gefäße in der Nähe des Aneurysmas und speziell in der Nähe der Öffnung des Aneurysmas weiterhin perfundiert werden, also mit Blut versorgt werden. Diese Ausführungsform hat sich ebenfalls besonders bei Aneurysmen mit benachbarten Seitenästen sowie bei kleineren Aneurysmen als geeignet erwiesen.

Die proximale Beabstandung ist vor allem in Hinblick auf die Arretierung am Führungsdraht aufgrund der offenen proximalen Zellen von Bedeutung. Wenn die proximalen Randzellen offen, d.h. unbespannt ausgeführt sind, kann bspw. das Verriegelungssystem der Anmelderin, die sog. "Crown-Sleeve" des Transportdrahts sicher eingekoppelt werden. Dafür genügt eine Obergrenze von 80% Bespannung. Ein Verbleib mit offenen Randzellen von 10% der Gesamtlänge L pro axialer Seite sollte ausreichend sein.

In einer Ausführungsform weist die Abdeckung auf einer Fläche von 100.000 µm² mindestens 10 Poren auf, die eine Größe von mindestens 15 µm² aufweisen. Im Zuge der Herstellung der Abdeckung lässt sich die Mindestgröße der Poren insbesondere durch die Prozessdauer des Elektrospinnens einstellen. Diese Kombination aus einer bestimmten Mindestanzahl an Poren und einer Mindestgröße dieser Poren hat sich in der Praxis als besonders förderlich für eine ausreichende Blutdurchlässigkeit der Abdeckung bei gleichzeitig guter Abdeckwirkung gezeigt.

Bevorzugt ist die Abdeckung durch ein Kunststoffmaterial, insbesondere durch ein Polymer, und vorzugsweise durch Polyurethan gebildet. Derartige Materialien sind besonders leicht und lassen sich gut in feinen Fäden durch ein elektrospinnendes Verfahren herstellen. Das Kunststoffmaterial ermöglicht es also einerseits eine besonders dünne und feinporige Abdeckung herzustellen. Andererseits weist das Kunststoffmaterial in sich bereits eine hohe Flexibilität auf, so dass eine hohe Komprimierbarkeit des medizinischen Sets erreicht wird. Alternativ kann die Abdeckung auch durch Polyethylen, durch Flurpolymere oder durch beispielsweise auf Polycarbonat basierenden, thermoplastischen Polyurethanen gebildet sein. Weiterhin kann beispielsweise alternativ oder ergänzend vorgesehen sein, dass Füllstoffe, wie z.B. anti-thrombogene Stoffe in die zuvor genannten Materialien der Abdeckung eingebettet sind, bevor mit diesen durch den Elektrospinning-Prozess die Abdeckung gebildet wird. Alternativ oder ergänzend ist die Abdeckung mit derartigen Stoffen, beispielsweise mit antithrombogenen Stoffen beschichtet. Hierzu ist dann die Oberfläche der Abdeckung insbesondere mit einer Nanobeschichtung versehen.

Vorzugsweise ist die Abdeckung durch ein Kunststoffmaterial, insbesondere ein Polymer, vorzugsweise Polyurethan, gebildet, das vorzugsweise eine Shore-Härte von wenigstens 80A, insbesondere wenigstens 90A, insbesondere wenigstens 55D, insbesondere wenigstens 65D, insbesondere wenigstens 75D aufweist. Diese Materialwerte haben sich für die Membranfunktion der Abdeckung als vorteilhaft erwiesen.

In einer weiteren Ausführungsform ist die Abdeckung auf einer Außenseite und/oder auf einer Innenseite der Gitterstruktur angeordnet. Die Gitterstruktur bildet in einer Konstellation, in der die Abdeckung auf der Außenseite der Gitterstruktur angeordnet ist eine Trägerstruktur, die eine ausreichende Radialkraft aufbringt, um die Abdeckung gegen eine Gefäßwand zu fixieren. Die Gitterstruktur stützt insoweit die außen angeordnete Abdeckung.

Alternativ oder zusätzlich kann die Abdeckung auf einer Innenseite der Gitterstruktur angeordnet sein. Insbesondere ist es möglich, dass die Gitterstruktur zwischen zwei Abdeckungen eingebettet ist, die jeweils durch ein elektrogesponnenes Gewebe gebildet sind. Die Gitterelemente der Gitterstruktur können insofern von dem elektrogesponnenen Gewebe vollständig ummantelt sein. Konkret kann vorgesehen sein, dass sich das elektrogesponnene Gewebe einer Abdeckung auf der Innenseite der Gitterstruktur in durch die Zellen der Gitterstruktur hindurcherstreckt und mit dem elektrogesponnenen Gewebe einer Abdeckung auf der Außenseite der Gitterstruktur verbunden ist. Die Gitterelemente, die die Zellen begrenzen, sind so auf allen Seiten von elektrogesponnenen Gewebe ummantelt.

Gemäß einer bevorzugten Ausgestaltung ist die Gitterstruktur aus Stegen gebildet, die einstückig, also monolithisch, miteinander verbunden sind und geschlossene, insbesondere rautenförmige Zellen begrenzen. Vorzugsweise weist die Gitterstruktur in Umfangsrichtung 3 bis 9, insbesondere 4 bis 6 hintereinander angeordnete Zellen auf, die einen umfangsseitigen Zellenring ausbilden.

Die Gitterstruktur kann somit grundsätzlich als einstückige Gitterstruktur ausgebildet sein. Insoweit ist es in bevorzugten Ausführungsformen vorgesehen, dass die Gitterelemente Stege bilden, die durch Stegverbinder einstückig miteinander gekoppelt sind (einstückige Gitterstruktur).

Es ist auch möglich, das die Gitterstruktur aus miteinander verflochtenen Drähten gebildet ist. Das Drahtgeflecht kann aus einem einzigen Draht bestehen, der an den Längsenden der Netzstruktur umgelenkt und zurückgeführt wird. Der Draht kann mit sich selbst verflochten werden, um die Netzstruktur zu bilden. Die Netzstruktur kann auch aus mehreren Drähten bestehen, die miteinander verflochten sind. Die mehreren Drähte können an einem axialen Längsende umgelenkt und zurückgeführt werden, während das gegenüberliegende axiale Längsende Drahtenden aufweisen kann, die offen sind. Es ist auch möglich, dass die miteinander verwebten Drähte offene Drahtenden an beiden axialen Längsenden aufweisen.

Die Gitterstruktur kann also ein Geflecht umfassen, das aus wenigstens einem Draht, insbesondere aus mehreren Drähten unter Bildung von Maschen geflochten ist, wobei die der Draht bzw. die Drähte an Überkreuzungspunkten relativ zueinander beweglich sind.

Das Embolisationsmittel kann wenigstens einen verformbaren Draht, insbesondere einen Coil, und/oder eine Embolisationsflüssigkeit umfassen. Bei der Embolisationsflüssigkeit handelt es sich konkret um eine Embolisationsflüssigkeit, die durch einen Mikrokatheter mit Außendurchmesser von höchstens 2Fr (Innendurchmesser höchstens 0.017" / 0,43mm) zuführbar ist.

Der Draht kann ein röntgensichtbares Kernmaterial und ein Mantelmaterial aus einer Formgedächtnislegierung aufweisen. Insbesondere ist vorgesehen, dass das Volumenverhältnis zwischen dem Kernmaterial, vorzugsweise Platin, und dem Volumen des gesamten Verbunddrahts zwischen 20% und 40%, insbesondere zwischen 25% und 35% ist. Während eine geflochtene Gitterstruktur sich durch eine besonders hohe Flexibilität, insbesondere Biegeflexibilität, auszeichnet, weist eine einstückige Gitterstruktur eine vergleichsweise dünne Wandstärke auf, so dass die Gitterstruktur den Blutfluss innerhalb eines Blutgefäßes weniger stark beeinflusst. Die Stege weisen weiterhin bevorzugt eine Dicke mit einem Wert im Bereich zwischen 30µm und 60µm auf. Die Zellen sind weiterhin jeweils durch insgesamt vier Stege begrenzt, wobei die Grundgeometrie der Zellen im Wesentlichen im bevorzugten Design rautenförmig ist. Insbesondere ist jede Zelle durch zwei Paar Stege begrenzt, wobei die Stege, die im Wesentlichen parallel zueinander oder gegenüberliegend und nicht direkt miteinander verbunden sind, ein Stegpaar bilden. Ein derartiges Design ist bereits in der Fig. 1 und den Absätzen [0041] bis [0046] der Patentschrift DE 10 2011 009 371 B3 der Anmelderin beschrieben, auf die insoweit verwiesen wird. Die Stege eines ersten Stegpaares weisen eine geringere Stegbreite auf als die Stege eines zweiten Stegpaares. Diese Anordnung der Stege mit unterschiedlichen Stegbreiten erhöht die Flexibilität der Netzstruktur und erleichtert so die Einführung der medizinischen Vorrichtung in menschliche Gefäße, insbesondere wenn diese starke Gefäßkrümmungen aufweisen. Die erhöhte Flexibilität verbessert die Anbringung an die Gefäßwand und verhindert so die Bildung von kongestiven Bereichen, die die Thrombose fördern. Die gute Flexibilität und die daraus resultierende gute Einführbarkeit im und/oder durch den Katheter ist besonders wichtig in Kombination mit einer biologischen Beschichtung, vorzugsweise Fibrin, vorzugsweise Fibrin, vorzugsweise Fibrin einschließlich Heparin.

Das Gewebe kann zusätzlich zu den durch Elektrospinnen gebildeten Poren zumindest bereichsweise durch weitere Poren perforiert sein, die im elektrogesponnenen Gewebe durch eine Bearbeitung des Gewebes, insbesondere durch Laserschneiden oder thermisches Aufweiten mittels Laser, ausgebildet sind. Dadurch wird eine gezielte und, falls gewünscht, bereichsweise Erhöhung der Porosität bzw. Vergrößerung der Poren nach dem Elektrospinning-Prozess erreicht. Z.B. können lasergeschnittene, definierte Poren bzw. mit dem Laserstrahl thermisch vergrößerte Poren auf dem gesamten Umfang oder auch nur auf einem Teil davon ausgebildet werden.

Das Gewebe ist vorzugsweise auf mindestens 25%, insbesondere auf mindestens 40%, insbesondere auf mindestens 50% des Umfangs der Gitterstruktur durch die weiteren Poren perforiert. Somit kann bspw. der Bereich gegenüber dem Aneurysma-Hals gezielt perforiert werden.

Das Gewebe kann auf mindestens 25%, insbesondere auf mindestens 40%, insbesondere auf mindestens 50% des Umfangs der Gitterstruktur frei von weiteren Poren sein. Mit anderen Worten wird ein Teil des Gewebes nicht nachbehandelt bzw. nachträglich perforiert. In diesem Teil des Gewebes werden keine weiteren Poren, zusätzlich zu den durch das Elektrospinnen ausgebildeten Poren in das Gewebe eingebracht. Das Gewebe besteht in diesem Bereich nur aus den durch das Elektrospinnen gebildeten Poren. Der frei von weiteren Poren ausgebildete Bereich des Gewebes kann im implantierten Zustand im Bereich des Aneurysma-Halses angeordnet sein. Dies kann beispielsweise dann gewünscht sein, wenn die Porosität des elektrogesponnenen Gewebes unverändert für die Behandlung des Aneurysmas vorteilhaft ist.

Eine Kombination von Bereichen aus unverändert elektrogesponnenem Gewebe und nachträglich perforiertem elektrogesponnenem Gewebe ist möglich.

Die weiteren Poren können ausgehend von der axialen Mitte der Gitterstruktur in beide axialen Richtungen ausgebildet sein. In einem weiteren Ausführungsbeispiel können zusätzliche Poren proximal- oder distalseitig innerhalb der Bespannung bzw. des Gewebes angeordnet sein.

Die Länge über die die weiteren Poren verteilt angeordnet sein können, entspricht mindestens 25% der axialen Länge der Abdeckung bzw. des Gewebes, insbesondere mindestens 30%, insbesondere mindestens 40%, insbesondere mindestens 50% der axialen Länge der Abdeckung bzw. des Gewebes.

Um die Durchströmung zu fördern, kann die Größe der weiteren Poren mindestens 50µm, insbesondere mindestens 100µm, insbesondere mindestens 200µm, insbesondere mindestens 300µm betragen.

Zur Geometrie der nachträglich hinzugefügten Porenvergrößerung wird allgemein ergänzt, dass die Form der weiteren Poren rund oder oval ausgeformt sein kann. Es ist auch möglich, dass die weiteren Poren kein erkennbares Formmuster aufweisen.

Die Abstände der weiteren Poren zueinander können in Bezug zum Durchmesser der weiteren Poren mindestens den 1-fachen Abstand, insbesondere mindestens 1,5-fachen Abstand, insbesondere mindestens 2-fachen Abstand, insbesondere mindestens 2,5-fachen Abstand betragen. Bei einem 1-fachen Abstand spricht dieser also dem Durchmesser einer weiteren Pore.

Bei einer besonders bevorzugten Ausführungsform ist die Umfangskontur der Abdeckung wenigstens abschnittsweise, insbesondere vollumfänglich, durch ein röntgensichtbares Mittel markiert. Dies kann beispielsweise durch röntgendichte Drähte erreicht werden, die in die Gitterstruktur entlang der Kontur der Abdeckung eingeflochten sind. Es ist auch möglich, die Kontur der Abdeckung durch eine Anreihung von röntgendichten Hülsen, beispielsweise Pt-Ir Hülsen oder gecrimpten C-Hülsen zu erreichen.

Die Lage der Bespannung bzw. des Gewebes ist somit unter Röntgenstrahlung so sichtbar, dass der Arzt die Vorrichtung sicher - auch in der richtigen Rotationslage - platzieren kann.

Das Gewebe kann als solches ein röntgensichtbares Mittel aufweisen. Bspw. können die Fäden des Gewebes mit einem röntgenundurchlässigen Material gefüllt sein, insb. mit mindestens 10% bis zu maximal 25% an röntgenundurchlässigen Material, z.B. Bariumsulfat BaSO₄. Die Grundfarbe der Fäden des Gewebes kann transparent sein, bei Hinzugabe von Bariumsulfat BaSO4 können diese weiß/ gelblich erscheinen.

Ein nebengeordneter Aspekt der Erfindung betrifft ein medizinisches System zur Behandlung von Aneurysmen mit einem medizinischen Set. Bei dem Set handelt es sich um das bereits vorstehend beschriebene medizinische Set. Das System umfasst ferner ein Zuführmittel, insbesondere einen Mikrokatheter, mit dem die Abdeckung zur Einbringung des Embolisationsmittels perforierbar ist.

Erfindungsgemäß weisen die Zellen der Gitterstruktur im expandierten Zustand einen Inkreisdurchmesser auf oder sind auf einen Inkreisdurchmesser aufweitbar, der wenigstens dem Außendurchmesser des Zuführmittels entspricht. Der Inkreisdurchmesser ist der Durchmesser des größtmöglichen Kreises, der sich in die Pore einschreiben lässt. Mit anderen Worten entspricht der Inkreisdurchmesser der Pore dem Außendurchmesser eines Zylinders, der sich gerade noch durch die Pore schieben lässt. Wenn das Zuführmittel, insbesondere der Katheter bei der Applikation nicht unter einem Winkel von 90° zur Pore der Abdeckung bzw. zur Zelle der Gitterstruktur ausgerichtet ist, entsteht eine ovale Öffnung, die mehr Fläche einnimmt.

Hierdurch wird insbesondere bei der vorstehend erläuterten Ausführungsform des medizinischen Systems sichergestellt, dass das Zuführmittels einfach in das Aneurysma durch die Abdeckung bzw. das Gewebe hindurch eingeführt werden kann.

Bevorzugt ist das Embolisationsmittel durch einen verformbaren Draht, insbesondere Coildraht, insbesondere durch einen bereits vorstehend erwähnten Coil, oder durch eine Flüssigkeit, z.B. durch ein Hydrogel gebildet. Derartige Ausbildungen des Embolisationsmittels haben sich im Hinblick auf eine Behandlung von Aneurysmen als besonders geeignet erwiesen.

Die im Hinblick auf das medizinische Set aufgeführten Vorteile und bevorzugten Ausgestaltungen sind sinngemäß auf das medizinische System zu übertragen und umgekehrt. Alle in Bezug auf das medizinische Set und in Bezug auf das medizinische System aufgeführten Dimensionsangaben gelten für einen expandierten Zustand der Gitterstruktur, sofern nicht etwas Anderes angegeben ist.

Die Erfindung wird anhand von Ausführungsbeispielen mit weiteren Einzelheiten unter Bezug auf die beigefügten schematischen Zeichnungen näher erläutert.

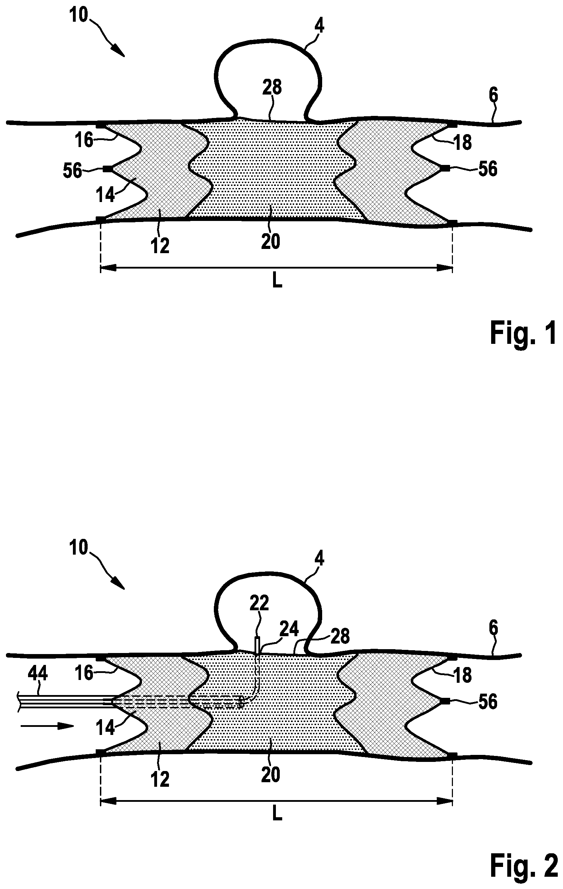

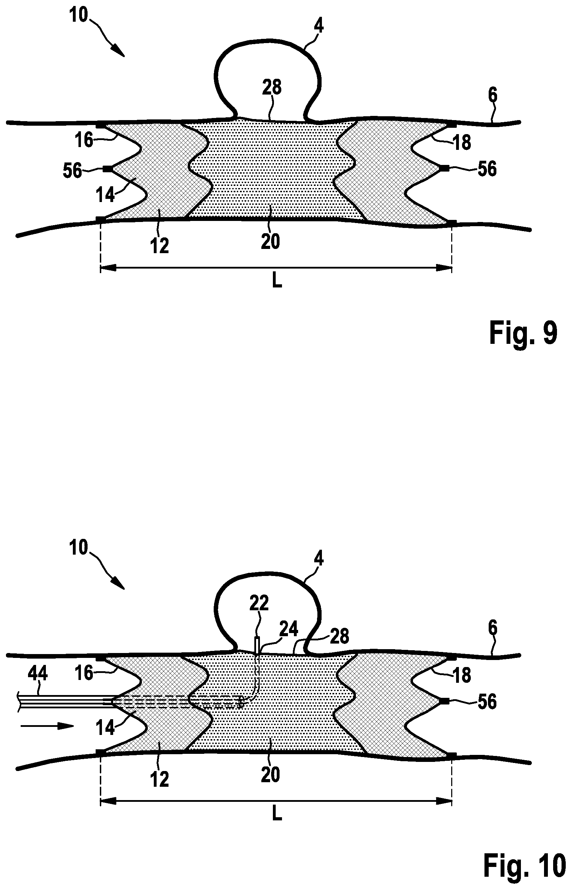

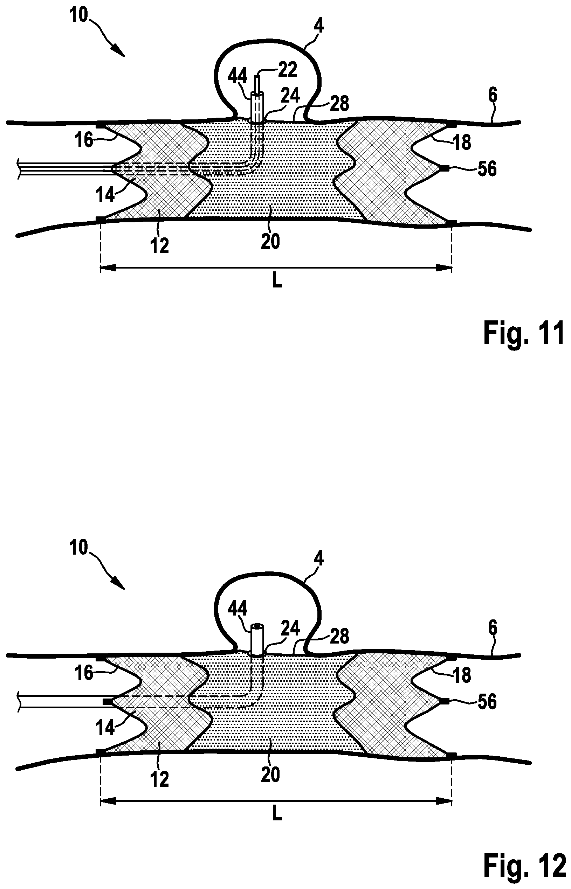

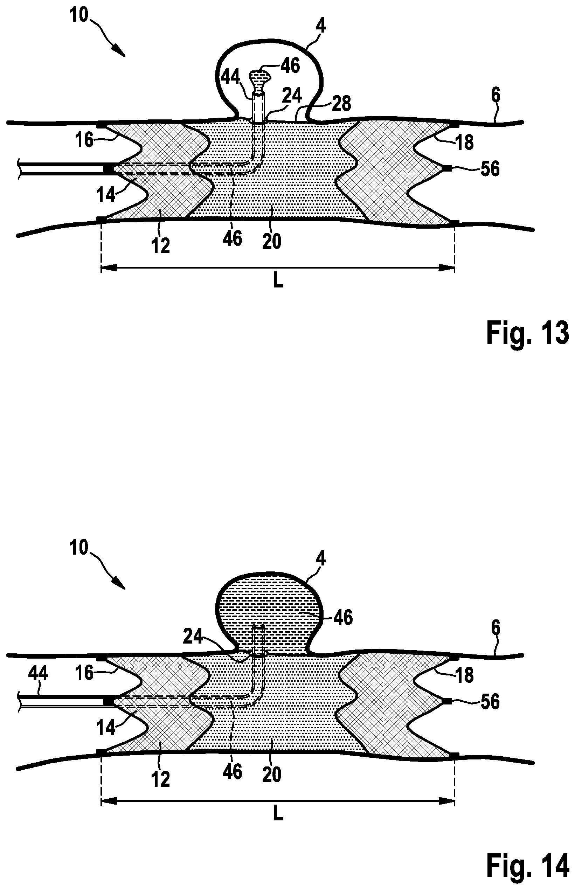

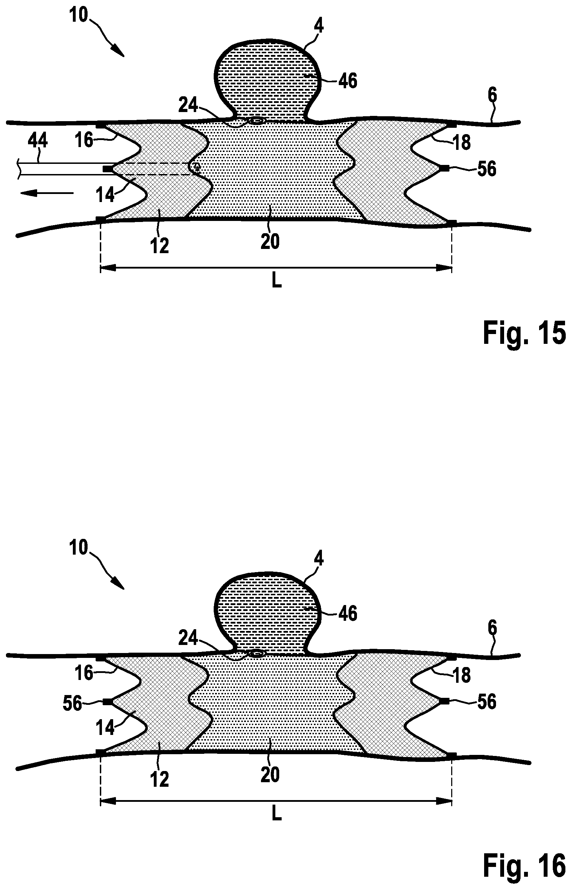

Dabei zeigen die Figuren 1 bis 8 den Einsatz eines medizinischen Sets nach einem erfindungsgemäßen Ausführungsbeispiel, wobei das Embolisationsmittel ein Coil ist. Die Figuren 9 bis 16 zeigen eine weitere Einsatzmöglichkeit des medizinischen Sets nach einem erfindungsgemäßen Ausführungsbeispiel, wobei das Embolisationsmittel ein Embolisat ist.

Konkret zeigen die Figuren:
- Fig.1: eine Seitenansicht einer Abdeckvorrichtung eines erfindungsgemäßen medizinischen Sets gemäß einer ersten Ausführungsform im implantierten Zustand (Schritt 1);
- Fig. 2: eine Seitenansicht der Abdeckvorrichtung gemäß Fig. 1 mit einem Zuführmittel und einem Führungsdraht, der die Abdeckung der Abdeckvorrichtung durchdringt (Schritt 2);
- Fig. 3: eine Seitenansicht der Abdeckvorrichtung gemäß Fig. 1, wobei das Zuführmittel die Abdeckung durchdringt (Schritt 3);
- Fig. 4: eine Seitenansicht der Abdeckvorrichtung gemäß Fig. 1, wobei der Führungsdraht zurückgezogen ist (Schritt 4);
- Fig. 5: eine Seitenansicht der Abdeckvorrichtung gemäß Fig. 1, wobei durch das Zuführmittel ein Coildraht vorgeschoben wird (Schritt 5);
- Fig. 6: eine Seitenansicht der Abdeckvorrichtung gemäß Fig. 1, wobei der Coildraht in das Aneurysma eingebracht wird (Schritt 6);
- Fig. 7: eine Seitenansicht der Abdeckvorrichtung gemäß Fig. 1, wobei das Zuführmittel aus dem Gefäß entfernt wird und die verbleibende Öffnung nach Entfernung des Zuführmittels stark verkleinert ist (Schritt 7);
- Fig. 8: eine Seitenansicht der Abdeckvorrichtung gemäß Fig. 1 im Endzustand, wobei die Abdeckung den im Aneurysma angeordneten Coildraht zurückhält und die verbleibende Öffnung nach Entfernung des Zuführmittels stark verkleinert ist (Schritt 8);
- Fig. 9: eine Seitenansicht einer Abdeckvorrichtung eines erfindungsgemäßen medizinischen Sets gemäß einer zweiten Ausführungsform im implantierten Zustand (Schritt 1);
- Fig. 10: eine Seitenansicht der Abdeckvorrichtung gemäß Fig. 9 mit einem Zuführmittel und einem Führungsdraht, der die Abdeckung der Abdeckvorrichtung durchdringt (Schritt 2);
- Fig. 11: eine Seitenansicht der Abdeckvorrichtung gemäß Fig. 9, wobei das Zuführmittel die Abdeckung durchdringt (Schritt 3);
- Fig. 12: eine Seitenansicht der Abdeckvorrichtung gemäß Fig. 9, wobei der Führungsdraht zurückgezogen ist (Schritt 4);
- Fig. 13: eine Seitenansicht der Abdeckvorrichtung gemäß Fig. 9, wobei durch das Zuführmittel eine Embolisationsflüssigkeit transportiert wird (Schritt 5);
- Fig. 14: eine Seitenansicht der Abdeckvorrichtung gemäß Fig. 9, wobei die Embolisationsflüssigkeit in das Aneurysma eingebracht wird (Schritt 6);
- Fig. 15: eine Seitenansicht der Abdeckvorrichtung gemäß Fig. 9, wobei das Zuführmittel aus dem Gefäß entfernt wird und die verbleibende Öff- nung nach Entfernung des Zuführmittels stark verkleinert ist (Schritt 7);
- Fig. 16: eine Seitenansicht der Abdeckvorrichtung gemäß Fig. 1 im Endzustand, wobei die Abdeckung die im Aneurysma angeordnete Embolisationsflüssigkeit zurückhält und die verbleibende Öffnung nach Entfernung des Zuführmittels stark verkleinert ist (Schritt 8);
- Fig. 17: eine Seitenansicht einer Abdeckvorrichtung eines erfindungsgemäßen medizinischen Sets gemäß einer weiteren Ausführungsform im implantierten Zustand und
- Fig. 18: eine Seitenansicht einer Abdeckvorrichtung eines erfindungsgemäßen medizinischen Sets gemäß einer weiteren Ausführungsform im implantierten Zustand.

In den Figuren sind gleichwirkende Teile mit den gleichen Bezugszeichen dargestellt.

Das in den Fig. 1 bis 8 schematisch dargestellte medizinische Set 2 dient zur Behandlung von Aneurysmen 4, bzw. allgemein von Gefäßmalformationen wie zum Beispiel Fisteln, und ist in Fig. 1 in einem innerhalb eines Gefäßes 6 angeordnetem Zustand gezeigt.

Das medizinische Set 2 weist eine durch einen nicht dargestellten Katheter hindurch an einen Behandlungsort 10 bewegbare Abdeckvorrichtung 12 auf. Bei den Behandlungsort 10 handelt es sich vorzugsweise um den Ort entlang des Gefäßes 6, an dem das Aneurysma 4 ausgebildet ist. Die Abdeckvorrichtung 12 dient dem permanenten Abdecken des Aneurysmas 4. Dies bedeutet, dass die Abdeckvorrichtung 12, nachdem diese vollständig aus dem Katheter entlassen wurde, nicht mehr in diesen zurückgezogen werden kann, sondern permanent im Gefäß verbleibt. Konkret handelt es sich bei der Abdeckvorrichtung 12 um ein permanentes Implantat, insbesondere um einen Stent.

Von der permanenten Abdeckvorrichtung abzugrenzen sind temporäre Abdeckvorrichtungen, die nach der Behandlung aus dem Gefäß wieder entfernt werden. Ein weiteres Unterscheidungsmerkmal der permanenten Abdeckvorrichtung ist die lösbare Verbindung mit dem Transportdraht, die erforderlich ist, um die Abdeckvorrichtung bzw. Gitterstruktur nach dem vollständigen Entlassen aus dem Zuführmittel vom Zuführsystem abkoppeln zu können. Dies ist bei temporären Abdeckvorrichtungen nicht der Fall, die fest mit dem Transportdraht zum Wiedereinziehen in das Zuführmittel verbunden sind.

Die Abdeckvorrichtung 12 umfasst eine selbstexpandierbare Gitterstruktur 14. Die Gitterstruktur 14 ist hierzu bevorzugt aus einem Formgedächtnismaterial gefertigt. Die Gitterstruktur 14 ist röhrchenförmig oder hohlzylinderförmig und am proximalen Längsende 16 und am distalen Längsende 18 offen. Dies bedeutet, dass die Gitterstruktur 14 an beiden Längsenden 16, 18 einen Strömungsquerschnitt aufweist, der frei von der Gitterstruktur 14 ist. Der Strömungsquerschnitt ist der quer zur Längsachse der Gitterstruktur 14 durchströmbare Querschnitt, der radial außen von dem Gefäß bzw. von der Gitterstruktur 14 begrenzt wird. Darin unterscheidet sich die permanent implantierbare Abdeckvorrichtung bzw. die Gitterstruktur 14 von der Gitterstruktur 14 einer temporär implantierbaren Abdeckvorrichtung, bei der die Gitterstruktur zumindest am proximalen Längsende trichterförmig in den Strömungsquerschnitt hineinragt. Bei der permanent implantierbaren Abdeckvorrichtung 12 liegt die Gitterstruktur 14 hingegen entlang der gesamten Länge an der Gefäßwand an und beaufschlagt diese mit einer Radialkraft.

Weiterhin ist die Gitterstruktur 14 wenigstens teilweise mit einer Abdeckung 20 versehen. Die Abdeckung 20 ist aus einem elektrogesponnene Gewebe hergestellt und bildet eine poröse Membran. Das elektrogesponnene Gewebe ist derart angepasst, dass dieses der Abdeckung eine Membranfunktion verleiht. Die poröse Membran ist derart ausgebildet, dass diese von dem Zuführmittel zur Applikation des Embolisationsmittels durchdrungen werden kann. Außerdem ist die Membran dazu angepasst, sich im durchdrungenen Zustand der Membran an den Außenumfang des Zuführmittels anzulegen.

Dies gilt für alle Abdeckungen 20 in dieser Anmeldung.

Für die Form der Abdeckung 20 bestehen verschiedene Möglichkeiten.

Die Abdeckung 20 kann beispielsweise, wie in den Figuren 1 bis 16 dargestellt, sich entlang einer Teillänge der Gitterstruktur 14 bzw. über einen Teil, insbesondere über höchstens 80%, insbesondere über höchstens 60% und speziell insbesondere über höchstens 40% der Gesamtlänge L der Gitterstruktur 16 erstrecken. Die Gesamtlänge L der Gitterstruktur ist in den Figuren eingezeichnet und erstreckt sich zwischen den äußersten axialen Längsenden entlang der Mittellinie der Gitterstruktur.

Hierdurch wird erreicht, dass sich die Abdeckung 20 im implantierten Zustand lediglich innerhalb des Bereichs der Öffnung 28 des Aneurysmas 4 befindet, sodass an das Aneurysma 4 angrenzende Zellen und/oder Seitengefäße nicht von der Abdeckung 20 abgedeckt werden. Somit können die Zellen und/oder die Seitengefäße weiterhin aufgrund der mit Zellen ausgebildeten Gitterstruktur 14 mit Blut und somit mit Nährstoffen versorgt werden.

Wie in den Figuren gut zu erkennen, ist die Abdeckung 20 im Wesentlichen mittig, d. h. im Bereich der axialen Mitte der Gitterstruktur 14 angeordnet. Mit anderen Worten sind die axialen Längsenden der Abdeckung 20 in etwa gleich weit vom proximalen und distalen Längsende 16, 18 der Gitterstruktur 14 entfernt bzw. beabstandet.

Die Kontur der Längsenden der Abdeckung 20, die sich in Umfangsrichtung der Gitterstruktur 14 erstreckt, entspricht im Wesentlichen der Kontur der proximalen und distalen Längsenden 16, 18 der Gitterstruktur. Dieses Merkmal wird sowohl im Zusammenhang mit den konkreten Ausführungsbeispielen, als auch allgemein im Zusammenhang mit weiteren Ausführungsbeispielen, die vorliegend nicht dargestellt sind, offenbart und beansprucht.

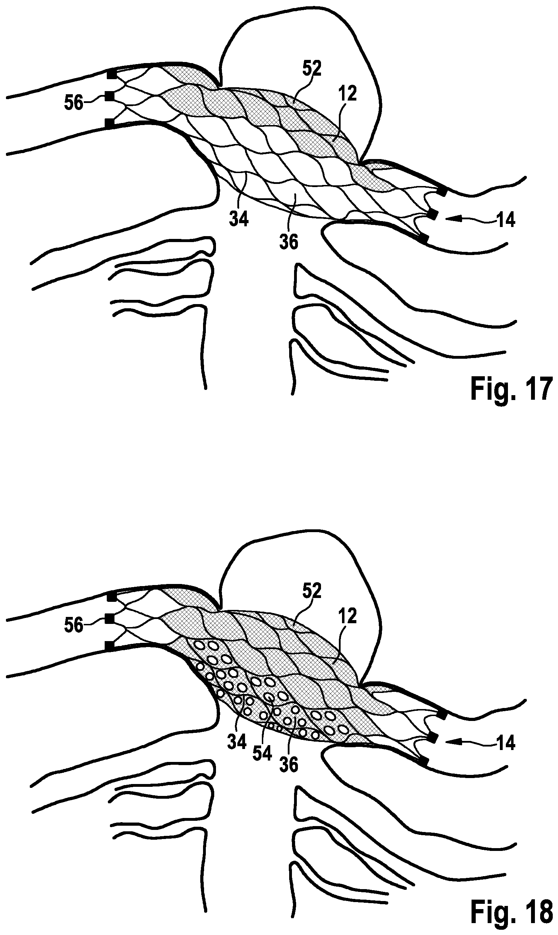

Bei den in den Figuren dargestellten Abdeckungen 20 erstreckt sich diese auf dem Gesamtumfang der Gitterstruktur 14. Es ist auch möglich, wie beispielsweise in den Figuren 17, 18 dargestellt, dass die Abdeckung 20 nur ein Teilsegment der Gitterstruktur 14 in Umfangsrichtung, also ein Winkelsegment, abdeckt.

Die Abdeckung 20 dient hierbei, insbesondere bei einem Platzieren eines Embolisationsmittels 40 innerhalb des Aneurysmas 4, dazu, dass das Embolisationsmittel 40 nach dem Platzieren nicht aus dem Aneurysma 4 entweichen kann, bis das Blut innerhalb des Aneurysmas 4 durch das Embolisationsmittel 40 geronnen und somit das Aneurysma 4 zuverlässig verschlossen ist.

Anhand der Figuren 1 bis 8 wird erläutert, wie das entsprechende Ausführungsbeispiel des medizinischen Sets umfassend die Abdeckvorrichtung 12 und das Embolisationsmittel 40 zur Behandlung eines Aneurysmas eingesetzt wird. Die Behandlung anderer Gefäßmalformationen ist entsprechend möglich.

In Fig. 1 ist die Abdeckvorrichtung 12 beispielsweise in der Form eines Stents im permanent implantierten Zustand, also nach kompletter Entlassung aus dem Zuführsystem dargestellt (Schritt 1). Die Abdeckvorrichtung 12 ist mit der Abdeckung 20 über der Aneurysmaöffnung 28 angeordnet. Die Abdeckung 20 überdeckt die Aneurysmaöffnung 28. Die Röntgenmarker 56 an den beiden axialen Längsenden 16, 18 markieren die Position der Abdeckvorrichtung 12.

Wie in Fig. 2 zu erkennen, wird in Schritt 2 ein Zuführmittel 44 in der Form eines Mikrokatheters durch das Lumen der Abdeckvorrichtung 12 eingeführt. Dazu ist in an sich bekannter Weise ein Führungsdraht 22 ebenfalls im Lumen der Abdeckvorrichtung 12 angeordnet, durch den das Zuführmittel 44 an den Behandlungsort geführt wird. Der Führungsdraht 22 durchdringt die Abdeckung 20 im Bereich der Aneurysmaöffnung 28, wie in Fig. 2 gut zu erkennen. Dabei kommt die Membranfunktion der Abdeckung 20 zum Tragen. Diese ermöglicht aufgrund der durch das elektrogesponnene Gewebe ausgebildeten Poren die Perforation bzw. Aufdehnung oder Aufweitung der Poren der Abdeckung durch den Führungsdraht 22. Der Führungsdraht 22 durchdringt das Material der Abdeckung 20, wobei die entsprechende Pore bzw. Poren, die der Führungsdraht 22 durchdringt, aufgeweitet wird bzw. werden. Dadurch wird die Öffnung 24 gebildet, durch die der Führungsdraht 22 ins Innere des Aneurysmas 4 geschoben wird.

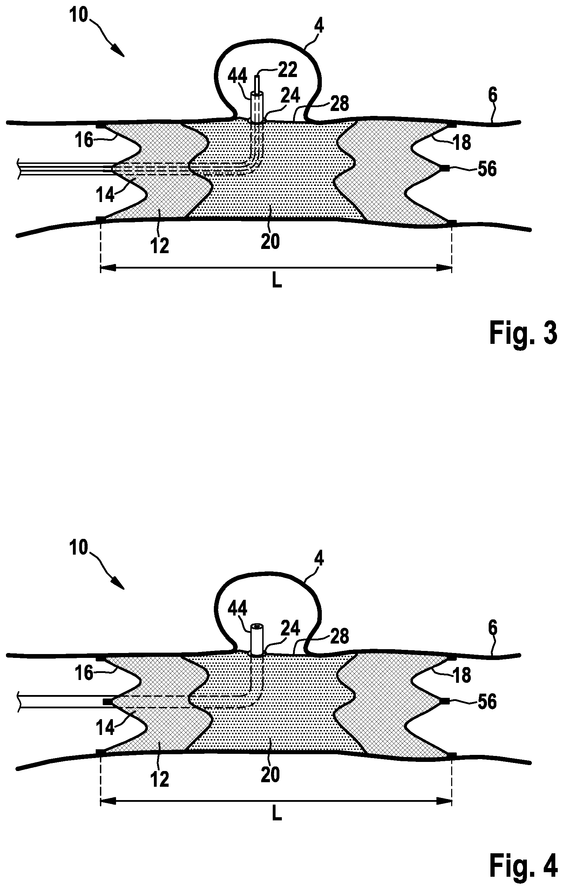

In Fig. 3 ist gezeigt, dass das Zuführmittel 44 über den Führungsdraht 22 weiter nach vorne in das Aneurysma 4 hinein geschoben wird. In diesem Zustand ragt das Zuführmittel 44, konkret das distale freie Ende des Zuführmittel 44 in das Aneurysma 4 hinein. Dabei durchdringt das Zuführmittel 44 die Abdeckung 20 im Bereich der bereits durch den Führungsdraht 22 gebildeten Öffnung 24. Die Öffnung 24 wird durch das Zuführmittel 44 auf den Außendurchmesser des Zuführmittels 44 weiter aufgeweitet. Die aufgeweitete Öffnung 24 legt sich eng an die Außenkontur des Zuführmittels 44 an und dichtet so das Aneurysma 4 gegen das Lumen der Abdeckvorrichtung 12 ab.

Dies wird durch die membranartige Ausbildung der Abdeckung 20 ermöglicht, konkret durch das elektrogesponnene Gewebe, das eine derartige Elastizität aufweist, dass das Material um die durchdrungene Pore/die durchdrungenen Poren herum bzw. die Öffnung 24 herum ausreichend elastisch verformt wird.

Gemäß Fig. 4 wird im Schritt 4 der Führungsdraht aus dem Zuführmittel 44 zurückgezogen, sodass das Zuführmittel 44 mit dem freien Ende im Aneurysma 4 platziert ist und ein freies Lumen für die Applikation des Embolisationsmittels 40 aufweist.

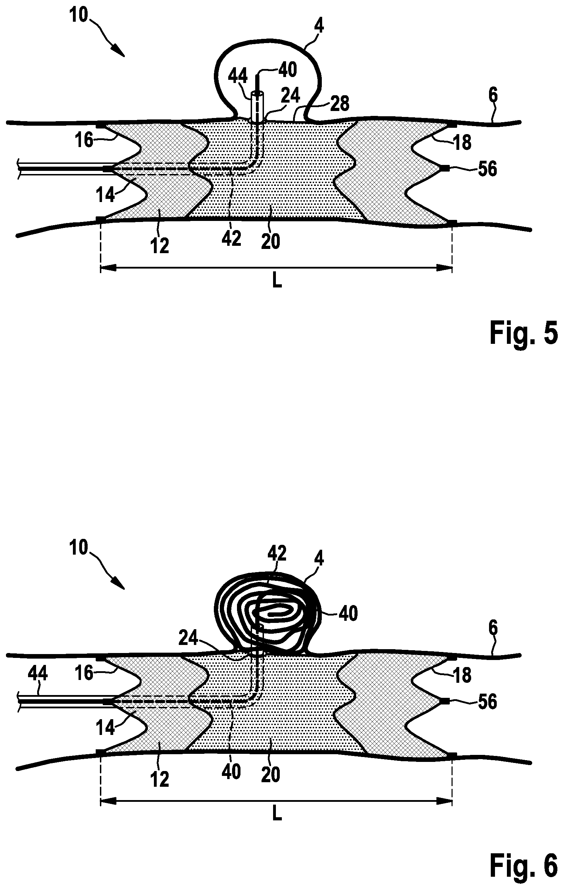

In Fig. 5 ist gezeigt, dass im Schritt 5 das Embolisationsmittel 40, konkret ein Coildraht 42 durch das Zuführmittel 44 und die Öffnung 24 in der Abdeckung 20 dem Aneurysma zugeführt wird.

Im Schritt 6, wie in Fig. 5 dargestellt, wird das Aneurysma 4 mit dem Embolisationsmittel 40 bzw. dem Coildraht 42 gefüllt. Bereits in diesem Zustand hält die Abdeckung 20 das Embolisationsmittel 40 im Aneurysma zurück. Außerdem dichtet die Abdeckung 20 im Bereich der Öffnung 24 das Aneurysma 4 gegen das Lumen der Abdeckvorrichtung 12 ab, da die Öffnung 24 eng am Außendurchmesser des Zuführmittel 44 anliegt. Ein Entweichen des Embolisationsmittels 40 wird dadurch bei der Applikation vermieden.

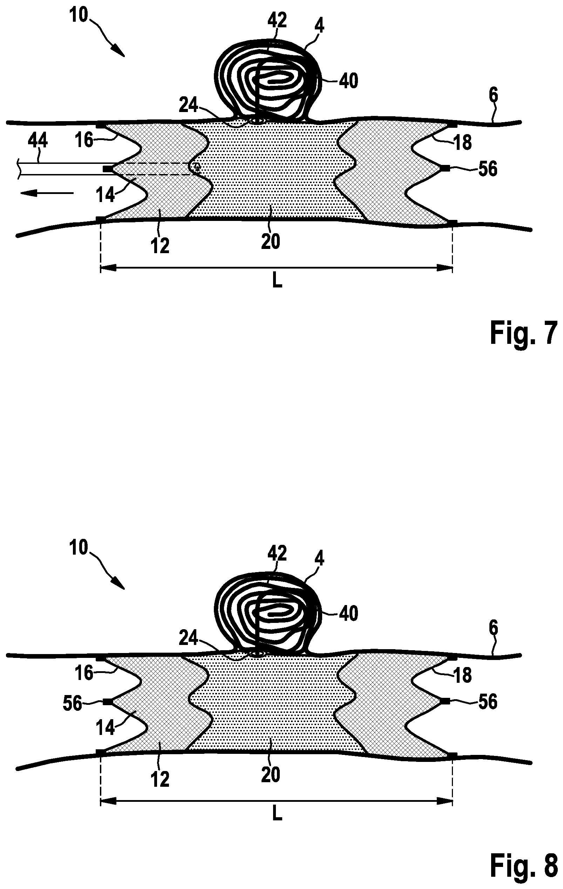

Nachdem das Aneurysma ausreichend mit dem Embolisationsmittel 40 gefüllt ist, wird das Zuführmittel 44 aus dem Lumen der Abdeckvorrichtung 12 entfernt, wie in Fig. 7 gezeigt (Schritt 7). In Fig. 7 ist gut zu erkennen, dass die Öffnung 24 sich zurückbildet. Die zurückgebildete Öffnung 24 weist einen kleineren Durchmesser als der Außendurchmesser des Zuführmittel 44 auf. Dadurch wird das Embolisationsmittel 40 im Aneurysma 4 sicher zurückgehalten. Es ist nicht zwingend erforderlich, dass sich die Öffnung 24 vollständig zurückbildet und die ursprüngliche Porengröße wieder einnimmt. Es genügt, wenn sich die Öffnung 24 soweit zurückbildet, dass durch die zurückgebildete Öffnung 24 kein Embolisationsmittel 40 in das Lumen der Abdeckvorrichtung 12 gelangen kann. Die durch das Zuführmittel zugeführten Coils / Hydrogele bzw. allgemein das zugeführte Embolisationsmittel, entsprechen bzw. entspricht ca. dem Innendurchmesser des Zuführmittels. Wenn die Öffnung nach Entfernen des Zuführmittels kleiner wird, kann das Embolisationsmittel, d.h. weder Coil noch Hydrogel durch die Öffnung entweichen.

Fig. 8 zeigt den Endzustand, nachdem das Zuführmittel 22 vollständig entfernt und die Abdeckvorrichtung 12 das Embolisationsmittel 40 im Aneurysma 4 zurückhält.

Es ist möglich, die Schritte 1 bis 8 in derselben Operation durchzuführen, d. h. zeitlich unmittelbar abfolgend und zusammenhängend. Es ist auch möglich, zunächst nur die Abdeckvorrichtung, wie in Fig. 1 gezeigt, zu implantieren, ohne zunächst die Schritte 2 bis 8 durchzuführen. Wenn sich herausstellt, dass der Behandlungserfolg noch nicht ausreichend eintritt, kann nachträglich die Behandlung des Aneurysmas mit einem Embolisationsmittel 40 problemlos durchgeführt werden. Das bedeutet, dass in einer zweiten, zeitlich späteren Operation die Schritte 2 bis 8 erfolgen. Das medizinische Set umfassend die Abdeckvorrichtung und das Embolisationsmittel ermöglicht damit eine flexible und zielgerichtete Behandlungsmethode von Aneurysmen.

Ein weiteres Ausführungsbeispiel ist in den Figuren 9 bis 16 dargestellt. Dieses Ausführungsbeispiel unterscheidet sich von dem Ausführungsbeispiel gemäß den Figuren 1 bis 8 darin, dass anstelle des Coildrahtes 42 ein anderes Embolisationsmittel 40 verwendet wird, nämlich eine Embolisationsflüssigkeit 46. Die Applikation der Embolisationsflüssigkeit 46 erfolgt wie die Applikation des Coildrahtes 42. Insofern wird auf die Erläuterungen zu den Figuren 1 bis 8 verwiesen.

Die Öffnung 24 ist bei diesem Ausführungsbeispiel so rückstellbar, dass nach dem Entfernen des Zuführmittel 44 eine ausreichend kleine Öffnung in der Abdeckung 20 verbleibt, durch die die Embolisationsflüssigkeit 46 nicht austreten kann. Auch hier gilt, dass sich die Öffnung 24 nicht vollständig verschließen muss, um den angestrebten Zweck zu erreichen.

Fig. 17 zeigt die Gitterstruktur 14 eines erfindungsgemäßen Ausführungsbeispiels im implantierten Zustand, wobei die Abdeckung 20 auf der Gitterstruktur 14 im Bereich des Aneurysma-Halses angeordnet ist und dieses überdeckt. Die Abdeckung 20 ist auf einem Teilumfang bzw. auf einem Winkelsegment der Gitterstruktur 14 angeordnet. In dem Beispiel bespannt das Gewebe bzw. die Abdeckung 20 in etwa den halben Umfang der Gitterstruktur 14 bzw. des Stents. Ein anderer Bespannungsgrad, also mehr oder weniger als der halbe Umfang der Gitterstruktur 14 ist möglich.

Dadurch ist eine Versorgung von dem Aneurysma 4 benachbarten Zellen und/oder Seitengefäßen weiterhin optimiert, da hierdurch bevorzugt lediglich die Öffnung 28 des Aneurysmas 4 abgedeckt ist und auch sich auf einer gleichen Höhe wie das Aneurysma 4 befindliche Zellen und/oder Seitengefäße weiterhin mit Blut und Nährstoffen versorgbar sind. Somit kann beispielsweise ein Seitengefäß, welches gegenüber des Aneurysmas 4 angeordnet ist, mit Blut versorgt werden.

Wie in Fig. 17 zu sehen, sind - im Unterschied zu Fig. 18 - keine weiteren Poren im Gewebe außer den durch das Elektrospinnen ausgebildeten Poren vorgesehen. Die Eigenschaften des Gewebes werden deshalb nur durch die beim Herstellungsprozess durch Elektrospinnen ausgebildeten Poren bestimmt.

Fig. 18 zeigt ein weiteres Ausführungsbeispiel der Erfindung, bei dem die Gitterstruktur 14, wie in Fig. 17, zur Behandlung eines Aneurysmas implantiert ist. Im Unterschied zu Fig. 17 ist die Abdeckung 20, insbesondere das Gewebe, vollumfänglich auf der Gitterstruktur 14 aufgebracht und zwar durch Elektrospinnen. Ein Teil der Abdeckung 20, konkret der dem Aneurysma-Hals gegenüberliegende Teil der Abdeckung 20, ist zusätzlich zu den beim Elektrospinnen ausgebildeten Poren 52 perforiert. Dies erfolgt durch eine Nachbehandlung des Gewebes, beispielsweise durch Laserschneiden oder thermisches Aufweiten mittels Laser. Die dadurch gebildeten weiteren Poren 54 im Gewebe sind größer als die durch das Elektrospinnen gebildeten Poren 52, wie in Fig. 18 zu erkennen. Bei dem Beispiel gemäß Fig. 18 sind pro Zelle vier weitere Poren 54 ausgebildet. Die Anzahl der weiteren Poren 54 kann variieren. Die weiteren Poren 54 sind im Unterschied zu den durch das Elektrospinnen gebildeten Poren 52 geometrisch definiert, beispielsweise rund. Dies wird durch das Laserschneiden oder thermische Aufweiten oder thermische Schmelzen mittels Laser ermöglicht.

Die zusätzliche Perforierung des Gewebes ermöglicht eine gezielte Beeinflussung der Durchströmbarkeit des Gewebes, beispielsweise um die Blutversorgung in Seitenästen zu verbessern, ohne dabei die Behandlung des Aneurysmas zu beeinträchtigen.

Wie in Fig. 17, 18 gut zu sehen ist, sind bei dem medizinischen Set 2 und speziell bei der Gitterstruktur 14 Röntgenmarker 56 vorgesehen. Die Röntgenmarker 56 sind an Zellenspitzen der randseitigen Zellen der Gitterstruktur 14 angeordnet. Konkret können die Röntgenmarker 56 als röntgensichtbare Hülsen, beispielsweise aus Platin oder Gold, gebildet sein, die auf die Zellenspitzen der randseitigen Zellen aufgecrimpt sind. Erkennbar ist in Fig. 17, 18 dass an jedem Längsende der Gitterstruktur 10 jeweils drei Röntgenmarker 56 angeordnet sind.

Die Abdeckvorrichtungen 14 gemäß Fig. 17 und Fig. 18 werden mit einem Embolisationsmittel 40 zu einem medizinischen Set kombiniert. Das Set kann wiederrum mit einem Zuführmittel 44 zu einem System kombiniert sein.

Die vorstehend genannten Ausführungsformen, insbesondere hinsichtlich eines sich Erstreckens der Abdeckung 20 entlang einer Länge L der Gitterstruktur 14 und hinsichtlich eines sich Erstreckens der Abdeckung 20 entlang eines Umfangs der Gitterstruktur 14 beliebig kombiniert ausgestaltet und kombiniert werden können. So ist beispielsweise auch eine Ausgestaltung möglich, bei der sich die Abdeckung 20 über die gesamte Länge L der Gitterstruktur erstreckt, sich jedoch zudem lediglich über einen Teil des Umfangs der Gitterstruktur 14 erstreckt.

### Bezugszeichenliste

- 2: medizinisches Set
- 4: Aneurysma
- 6: Gefäß
- 8: frei
- 10: Behandlungsort
- 12: Abdeckvorrichtung
- 14: Gitterstruktur
- 16: proximales Längsende
- 18: distales Längsende
- 20: Abdeckung
- 22: Führungsdraht
- 24: Öffnung
- 26: frei
- 27: Umfangsfläche
- 28: Öffnung des Aneurysmas
- 30: frei
- 32: frei
- 34: Stege
- 36: Zelle
- 40: Embolisationsmittel
- 42: Coildraht
- 44: Zuführmittel
- 46: Embolisationsflüssigkeit
- 48: frei
- 50: frei
- 52: Pore
- 54: weitere Poren
- 56: Röntgenmarker
- L: Länge

## Patentansprüche

1. Medizinisches Set zur Behandlung von Gefäßmalformationen, insbesondere Aneurysmen und/oder Fisteln, mit
- einer permanent implantierbaren Abdeckvorrichtung (12), insbesondere Stent, zum Abdecken der Gefäßmalformation, wobei die Abdeckvorrichtung (12) eine röhrchenförmige, selbstexpandierbare Gitterstruktur (14) und eine Abdeckung (20) aus einem elektrogesponnenen Gewebe aufweist, wobei die Abdeckung (20) mit der Gitterstruktur (14) verbunden ist und zumindest teilweise die Gitterstruktur (14) überdeckt, um im implantierten Zustand über der Gefäßmalformation platziert zu werden, und
- einem Embolisationsmittel (40), das durch ein Zuführmittel (44) im implantierten Zustand zur Behandlung der Gefäßmalformation applizierbar ist,
wobei die Abdeckung (20) eine poröse Membran bildet,
**dadurch gekennzeichnet, dass**
die Gitterstruktur (14) Zellen (36) oder Maschen aufweist, die im expandierten Zustand einen Inkreisdurchmesser (D) aufweisen oder auf einen Inkreisdurchmesser (D) aufweitbar sind, der wenigstens dem Außendurchmesser (A) des Zuführmittels entspricht, und die Abdeckung (20) von dem Zuführmittel (44) zur Applikation des Embolisationsmittels (40) durchdringbar und dazu angepasst ist, sich im durchdrungenen Zustand an den Außenumfang des Zuführmittels (44) anzulegen, wobei die poröse Membran dazu angepasst ist, eine durch das Zuführmittel beim Durchdringen der Membran gebildete Öffnung (24) nach dem Entfernen des Zuführmittels zumindest teilweise zurückzubilden, und wobei das Zuführmittel einen distalen Außendurchmesser von höchstens 0,7 mm aufweist.

2. Medizinisches Set nach Anspruch 1,
**dadurch gekennzeichnet, dass**
die poröse Membran der Abdeckung (20) dazu angepasst ist, die Öffnung (24) auf höchstens 80%, insbesondere höchstens 60%, insbesondere höchstens 40%, insbesondere höchstens 20% des Durchmessers des Zufuhrmittels zurückzubilden.

3. Medizinisches Set nach einem der vorhergehenden Ansprüche,
**dadurch gekennzeichnet, dass**
die Abdeckung (20) derart geringfügig porös ist, sodass sie im implantierten Zustand das applizierte Embolisationsmittel zurückhält, und/oder dass
die Abdeckung (20) eine Porosität von höchstens 70%, insbesondere höchstens 50%, insbesondere höchstens 40%, insbesondere höchstens 30% aufweist, und/oder dass
die Abdeckung (20) eine Porosität von wenigstens 5%, insbesondere wenigstens 10%, insbesondere wenigstens 20%, insbesondere wenigstens 25%, insbesondere wenigstens 30%, insbesondere wenigstens 40%, insbesondere wenigstens 45%, aufweist.

4. Medizinisches Set nach einem der vorhergehenden Ansprüche,
**dadurch gekennzeichnet, dass**
sich die Abdeckung (20) über den gesamten Umfang der Gitterstruktur (14) erstreckt oder dass
sich die Abdeckung (20) über einen Teil, insbesondere höchstens 70%, insbesondere höchstens 60%, insbesondere höchstens 50%, insbesondere höchstens 40%, insbesondere höchstens 30%, eines Umfangs der Gitterstruktur (14) erstreckt.

5. Medizinisches Set nach einem der vorhergehenden Ansprüche,
**dadurch gekennzeichnet, dass**
sich die Abdeckung (20) über höchstens 80%, insbesondere über höchstens 60%, insbesondere über höchstens 40%, der Länge (L) der Gitterstruktur (14) erstreckt, wobei die Abdeckung (20) vom distalen Ende (18) und/oder vom proximalen Ende der Gitterstruktur (14) beabstandet ist.

6. Medizinisches Set nach einem der vorhergehenden Ansprüche,
**dadurch gekennzeichnet, dass**
die Abdeckung (20) auf einer Fläche von 100.000 µm² mindestens 10 Poren (52) umfasst, die eine Größe von mindestens 15 µm² aufweisen und/oder dass
das elektrogesponnene Gewebe der Abdeckung (20) Fäden mit einer Fadendicke von höchstens 2 µm, insbesondere höchstens 1,5 µm, insbesondere höchstens 1 µm, und mit einer Fadendicke von wenigstens 0,3 µm aufweist, und/oder dass
die Abdeckung (20) durch ein Kunststoffmaterial, insbesondere ein Polymer, vorzugsweise Polyurethan, gebildet ist, das vorzugsweise eine Shore-Härte von wenigstens 80A, insbesondere wenigstens 90A, insbesondere wenigstens 55D, insbesondere wenigstens 65D, insbesondere wenigstens 75D aufweist.

7. Medizinisches Set nach einem der vorhergehenden Ansprüche,
**dadurch gekennzeichnet, dass**
die Abdeckung (20) auf einer Außenseite und/oder einer Innenseite der Gitterstruktur (14) angeordnet ist und/oder dass
das Embolisationsmittel (40) wenigstens einen verformbaren Draht (42), insbesondere einen Coil, und/oder eine Embolisationsflüssigkeit umfasst.

8. Medizinisches Set nach einem der vorhergehenden Ansprüche,
**dadurch gekennzeichnet, dass**
die Gitterstruktur (14) aus Stegen (34) gebildet ist, die einstückig miteinander verbunden sind und geschlossene, insbesondere rautenförmige, Zellen (36) begrenzen, oder dass
die Gitterstruktur (14) ein Geflecht umfasst, das aus wenigstens einem Draht, insbesondere aus mehreren Drähten unter Bildung von Maschen geflochten ist, wobei die der Draht bzw. die Drähte an Überkreuzungspunkten relativ zueinander beweglich sind.

9. Medizinisches Set nach einem der vorhergehenden Ansprüche,
**dadurch gekennzeichnet, dass**
das Gewebe zusätzlich zu den durch Elektrospinnen gebildeten Poren (52) zumindest bereichsweise durch weitere Poren (54) perforiert ist, die im elektrogesponnenen Gewebe durch eine Bearbeitung des Gewebes, insbesondere durch Laserschneiden oder thermisches Aufweiten mittels Laser, ausgebildet sind, insbesondere wobei
das Gewebe auf mindestens 25%, insbesondere auf mindestens 40%, insbesondere auf mindestens 50% des Umfangs der Gitterstruktur (10) durch die weiteren Poren perforiert ist.

10. Medizinisches Set nach Anspruch 16 oder 17,
**dadurch gekennzeichnet, dass**
das Gewebe auf mindestens 25%, insbesondere auf mindestens 40%, insbesondere auf mindestens 50%, des Umfangs der Gitterstruktur (10) frei von weiteren Poren ist und/oder dass die weiteren Poren ausgehend von der axialen Mitte der Gitterstruktur (10) in beide axialen Richtungen ausgebildet sind.

11. Medizinisches Set nach einem der Ansprüche 16 bis 19,
**dadurch gekennzeichnet, dass**
die Größe der weiteren Poren mindestens 50µm, insbesondere mindestens 100µm, insbesondere mindestens 200µm, insbesondere mindestens 300µm beträgt.

12. Medizinisches Set nach einem der Ansprüche 16 bis 19,
**dadurch gekennzeichnet, dass**
die Abstände der weiteren Poren zueinander in Bezug zum Durchmesser der weiteren Poren mindestens den 1-fachen Abstand, insbesondere mindestens 1,5-fachen Abstand, insbesondere mindestens 2-fachen Abstand, insbesondere mindestens 2,5-fachen Abstand betragen.

13. Medizinisches Set nach einem der vorhergehenden Ansprüche,
**dadurch gekennzeichnet, dass**
die Umfangskontur der Abdeckung (20) wenigstens abschnittsweise, insbesondere vollumfänglich, durch ein röntgensichtbares Mittel markiert ist und/oder dass
das Gewebe als solches ein röntgensichtbares Mittel aufweist.

14. Medizinisches System zur Behandlung von Gefäßmalformationen mit einem medizinischen Set nach einem der vorhergehenden Ansprüche, und einem Zuführmittel, mit dem die Abdeckung (20) zur Einbringung des Embolisationsmittels durchdringbar ist.

15. Permanent implantierbare Abdeckvorrichtung (12), insbesondere Stent, zur Behandlung von Gefäßmalformationen, insbesondere Aneurysmen und/oder Fisteln, insbesondere zum Abdecken der Gefäßmalformation, wobei die Abdeckvorrichtung (12) eine röhrchenförmige, selbstexpandierbare Gitterstruktur (14) und eine Abdeckung (20) aus einem elektrogesponnenen Gewebe aufweist, wobei die Abdeckung (20) mit der Gitterstruktur (14) verbunden ist und zumindest teilweise die Gitterstruktur (14) überdeckt, um im implantierten Zustand über der Gefäßmalformation platziert zu werden, wobei die Abdeckung (20) eine poröse Membran bildet, die von einem Zuführmittel (44) zur Applikation eines Embolisationsmittels (40) durchdringbar und dazu angepasst ist, sich im durchdrungenen Zustand an den Außenumfang des Zuführmittels (44) anzulegen, wobei die poröse Membran der Abdeckung (20) dazu angepasst ist, eine durch das Zuführmittel beim Durchdringen der Membran gebildete Öffnung (24) nach dem Entfernen des Zuführmittels auf höchstens 80%, insbesondere höchstens 60%, insbesondere höchstens 40%, insbesondere höchstens 20% des Durchmessers des Zufuhrmittels zurückzubilden und wobei die Abdeckung (20) auf einer Fläche von 100.000 µm² mindestens 10 Poren (52) umfasst, die eine Größe von mindestens 15 µm² aufweisen.

## Claims

1. A medical kit for the treatment of vascular malformations, in particular aneurysms and/or fistulas, with:
- a permanently implantable covering device (12), in particular a stent, for covering the vascular malformation, wherein the covering device (12) has a tubular, self-expandable mesh structure (14) and a covering (20) formed from an electrospun fabric, wherein the covering (20) is connected to the mesh structure (14) and at least partially covers the mesh structure (14) in order to be placed over the vascular malformation in the implanted state, and
- an embolization means (40) which, in the implanted state, is appliable by a delivery means (44) for the treatment of the vascular malformation,
wherein the covering (20) forms a porous membrane, **characterized in that**
the mesh structure (14) has cells (36) or meshes which, in the expanded state, have an inscribed diameter (D) or are expandable to an inscribed diameter (D) which corresponds to at least the external diameter (A) of the delivery means, and the covering (20) is penetrable by the delivery means (44) for the application of the embolization means (40) and is adapted to lie against the outer circumference of the delivery means (44) in the penetrated state, wherein the porous membrane is adapted to at least partially contract an opening (24) formed by the delivery means when the membrane is penetrated after the removal of the delivery means, and wherein the delivery means has a distal external diameter of at most 0.7 mm.

2. The medical kit as claimed in claim 1,
**characterized in that**
the porous membrane of the covering (20) is adapted to contract the opening (24) by at most 80%, in particular at most 60%, in particular at most 40%, in particular at most 20% of the diameter of the delivery means.

3. The medical kit as claimed in any of the preceding claims,
**characterized in that**
the covering (20) is slightly porous, in a manner such that in the implanted state, it retains the applied embolization means, and/or **in that**
the covering (20) has a porosity of at most 70%, in particular at most 50%, in particular at most 40%, in particular at most 30%, and/or **in that**
the covering (20) has a porosity of at least 5%, in particular at least 10%, in particular at least 20%, in particular at least 25%, in particular at least 30%, in particular at least 40%, in particular at least 45%.

4. The medical kit as claimed in any of the preceding claims,
**characterized in that**
the covering (20) extends over the entire circumference of the mesh structure (14), or **in that**
the covering (20) extends over a portion, in particular at most 70%, in particular at most 60%, in particular at most 50%, in particular at most 40%, in particular at most 30%, of a circumference of the mesh structure (14).

5. The medical kit as claimed in any of the preceding claims,
**characterized in that**
the covering (20) extends over at most 80%, in particular over at most 60%, in particular over at most 40% of the length (L) of the mesh structure (14), wherein the covering (20) is at a distance from the distal end (18) and/or from the proximal end of the mesh structure (14).

6. The medical kit as claimed in any of the preceding claims,
**characterized in that**
the covering (20) has at least 10 pores (52) with a size of at least 15 µm² over an area of 100000 µm², and/or **in that**
the electrospun fabric of the covering (20) has filaments with a filament thickness of at most 2 µm, in particular at most 1.5 µm, in particular at most 1 µm, and with a filament thickness of at least 0.3 µm, and/or in that
the covering (20) is formed by a plastic material, in particular a polymer, preferably polyurethane, which preferably has a Shore hardness of at least 80A, in particular at least 90A, in particular at least 55D, in particular at least 65D, in particular at least 75D.

7. The medical kit as claimed in any of the preceding claims,
**characterized in that**
the covering (20) is disposed on an outside and/or on an inside of the mesh structure (14), and/or **in that** the embolization means (40) comprises at least one deformable wire (42), in particular a coil, and/or a liquid embolic.

8. The medical kit as claimed in any of the preceding claims,
**characterized in that**
the mesh structure (14) is formed from struts (34) which are connected together in one piece and delimit closed, in particular diamond-shaped cells (36), or **in that** the mesh structure (14) comprises a braid which is braided from at least one wire, in particular from a plurality of wires with the formation of meshes, wherein the wire or the wires are movable relative to each other at points of intersection.

9. The medical kit as claimed in any of the preceding claims,
**characterized in that**
in addition to the pores (52) formed by electrospinning, the fabric is perforated, at least in regions, by further pores (54) which are formed in the electrospun fabric by processing the fabric, in particular by laser cutting or thermal widening by means of a laser, in particular wherein
the fabric is perforated by the further pores over at least 25%, in particular over at least 40%, in particular over at least 50% of the circumference of the mesh structure (10).

10. The medical kit as claimed in claim 16 or claim 17, **characterized in that**
the fabric is free from further pores over at least 25%, in particular over at least 40%, in particular over at least 50% of the circumference of the mesh structure (10) and/or **in that** the further pores are formed in both axial directions outwards from the axial centre of the mesh structure (10).

11. The medical kit as claimed in any of claims 16 to 19, **characterized in that**
the size of the further pores is at least 50 µm, in particular at least 100 µm, in particular at least 200 µm, in particular at least 300 µm.

12. The medical kit as claimed in any of claims 16 to 19, **characterized in that**
the distances between the further pores in relation to the diameter of the further pores are at least 1 time the distance, in particular at least 1.5 times the distance, in particular at least 2 times the distance, in particular at least 2.5 times the distance.

13. The medical kit as claimed in any of the preceding claims,
**characterized in that**
the circumferential contour of the covering (20) is marked by a radiopaque means, at least in sections, in particular entirely circumferentially, and/or **in that** the fabric per se has a radiopaque means.

14. A medical system for the treatment of vascular malformations with a medical set as claimed in any of the preceding claims, and a delivery means, with which the covering (20) is penetrable in order to introduce the embolization means.

15. A permanently implantable covering device (12), in particular a stent, for the treatment of vascular malformations, in particular aneurysms and/or fistulas, in particular in order to cover the vascular malformation, wherein the covering device (12) has a tubular, self-expandable mesh structure (14) and a covering (20) formed from an electrospun fabric, wherein the covering (20) is connected to the mesh structure (14) and at least partially covers the mesh structure (14) in order to be placed over the vascular malformation in the implanted state, wherein the covering (20) forms a porous membrane which is penetrable by a delivery means (44) for the application of an embolization means (40) and is adapted to lie against the outer circumference of the delivery means (44) in the penetrated state, wherein the porous membrane of the covering (20) is adapted so as to contract an opening (24) formed by the delivery means upon penetration of the membrane by at most 80%, in particular at most 60%, in particular at most 40%, in particular at most 20% of the diameter of the delivery means after removal of the delivery means, and wherein the covering (20) comprises at least 10 pores (52) which have a size of at least 15 µm² over an area of 100000 µm².

## Revendications

1. Ensemble médical pour le traitement de malformations vasculaires, en particulier d'anévrismes et/ou de fistules, avec
- un dispositif de recouvrement (12) implantable de façon permanente, en particulier un stent, pour couvrir la malformation vasculaire, le dispositif de recouvrement (12) présentant une structure en treillis tubulaire auto-expansible (14) et un couvercle (20) composé d'un tissu électrofilé, le couvercle (20) étant relié à la structure en treillis (14) et recouvrant au moins partiellement la structure en treillis (14) pour être placé à l'état implanté sur la malformation vasculaire, et
- un agent d'embolisation (40) qui est applicable à l'état implanté au moyen d'un moyen d'alimentation (44) pour le traitement des malformations vasculaires,
le couvercle (20) formant une membrane poreuse,
**caractérisé en ce que** la structure en treillis (14) présente des cellules (36) ou des mailles qui, à l'état déployé, présentent un diamètre interne (D) ou sont expansables jusqu'à un diamètre interne (D) correspondant au moins au diamètre externe (A) du moyen d'alimentation (44), et le couvercle (20) est traversable par le moyen d'alimentation (40) pour l'application du moyen d'embolisation et étant conçu pour s'appliquer à l'état traversé sur la circonférence extérieure du moyen d'alimentation (44), la membrane poreuse étant conçue pour former une ouverture (24) formée par le moyen d'alimentation lorsqu'il traverse la membrane au moins partiellement après le retrait du moyen d'alimentation, de et le moyen d'alimentation ayant un diamètre externe distal ne dépassant pas 0,7 mm.

2. Ensemble médical selon la revendication 1,
**caractérisé en ce que** la membrane poreuse du couvercle (20) est adaptée à l'ouverture (24) à au plus 80 %, en particulier à au plus 60 %, en particulier à au plus 40 %, en particulier à au plus 20 % du diamètre du moyen d'alimentation.

3. Ensemble médical selon l'une quelconque des revendications précédentes,
**caractérisé en ce que** le couvercle (20) est légèrement poreux au point de retenir l'agent embolisant appliqué à l'état implanté, et/ou que le couvercle (20) présente une porosité d'au plus 70 %, en particulier d'au plus 50 %, en particulier d'au plus 40 %, en particulier d'au plus 30 %, et/ou
que le couvercle (20) présente une porosité d'au moins 5 %, en particulier d'au moins 10 %, en particulier d'au moins 20 %, en particulier d'au moins 25 %, en particulier d'au moins 30 %, en particulier d'au moins 40 %, en particulier d'au moins 45 %.

4. Ensemble médical selon l'une des revendications précédentes,
**caractérisé en ce que** le couvercle (20) s'étend sur toute la périphérie de la structure en treillis (14) ou que le couvercle (20) s'étend sur une partie, en particulier au plus 70 %, en particulier au plus 60 %, en particulier au plus 50 %, en particulier au plus 40 %, en particulier au plus 30 %, d'une circonférence de la structure en treillis (14).

5. Ensemble médical selon l'une quelconque des revendications précédentes, **caractérisé en ce que** le couvercle (20) s'étend sur au plus 80 %, en particulier sur au plus 60 %, en particulier sur au plus 40 %, de la longueur (L) de la structure en treillis (14), le couvercle (20) étant espacé de l'extrémité distale (18) et/ou de l'extrémité proximale de la structure en treillis (14).

6. Ensemble médical selon l'une quelconque des revendications précédentes,
**caractérisé en ce que** le couvercle (20) comprend, sur une surface de 100 000 µm², au moins 10 pores (52) ayant une taille d'au moins 15 µm² et/ou
que le tissu électrofilé du couvercle (20) présente des fils d'une épaisseur de fil d'au plus 2 µm, en particulier d'au plus 1,5 µm, en particulier d'au plus 1 µm, et une épaisseur de fil d'au moins 0,3 µm, et/ou
que le couvercle (20) est formé d'une matière plastique, en particulier d'un polymère, de préférence du polyuréthane, qui présente de préférence une dureté Shore d'au moins 80 A, en particulier d'au moins 90 A, en particulier d'au moins 55 D, en particulier d'au moins 65 D, en particulier d'au moins 75 **D.**

7. Ensemble médical selon l'une quelconque des revendications précédentes,
**caractérisé en ce que** le couvercle (20) est disposé sur une face extérieure et/ou une face intérieure de la structure grillagée (14) et/ou
que l'agent d'embolisation (40) comprend au moins un fil déformable (42), en particulier un serpentin, et/ou un fluide d'embolisation.

8. Ensemble médical selon l'une des revendications précédentes,
**caractérisé en ce que** la structure en treillis (14) est formée de nervures (34) qui sont reliées les unes aux autres d'un seul tenant et qui limitent des cellules fermées (36), en particulier en forme de losange, ou
que la structure en treillis (14) comporte un treillis tressé à partir d'au moins un fil, en particulier de plusieurs fils, en formant des mailles, le fil ou les fils étant mobiles les uns par rapport aux autres à des points de croisement.

9. Ensemble médical selon l'une des revendications précédentes,
**caractérisé en ce que** le tissu est en outre perforé par des pores (52) constitués par électrofilage, du moins par endroits, par d'autres pores (54) qui sont formés dans le tissu électrofilé par traitement du tissu, en particulier par découpe au laser ou dilatation thermique au laser,
le tissu est en particulier perforé sur au moins 25 %, en particulier au moins 40 %, en particulier au moins 50 % de la circonférence de la structure en treillis (10) par les autres pores.

10. Ensemble médical selon la revendication 16 ou 17, **caractérisé en ce que** le tissu est exempt d'autres pores sur au moins 25 %, en particulier au moins 40 %, en particulier au moins 50 %, de la circonférence de la structure en treillis (10) et/ou que les pores supplémentaires sont formés dans les deux directions axiales en partant du centre axial de la structure en treillis (10).

11. Ensemble médical selon l'une des revendications 16 à 19,
**caractérisé en ce que** la taille des pores supplémentaires est d'au moins 50 µm, en particulier d'au moins 100 µm, en particulier d'au moins 200 µm, en particulier d'au moins 300 µm.

12. Ensemble médical selon l'une des revendications 16 à 19,
**caractérisé en ce que** les distances mutuelles entre les pores supplémentaires par rapport au diamètre des pores supplémentaires sont au moins égales à 1 fois la distance l, en particulier au moins 1,5 fois la distance, en particulier au moins 2 fois la distance, en particulier moins 2,5 fois la distance.

13. Ensemble médical selon l'une des revendications précédentes,
**caractérisé en ce que** le contour périphérique du couvercle (20) est marqué au moins partiellement, en particulier complètement, par un moyen visible aux rayons X et/ou
que le tissu en tant que tel contient un moyen visible aux rayons X.

14. Système médical pour le traitement de malformations vasculaires avec un ensemble médical selon l'une quelconque des revendications précédentes, et un moyen d'alimentation grâce avec lequel le couvercle (20) est traversable pour l'introduction de l'agent embolisant.

15. Dispositif de recouvrement (12) implantable à demeure, en particulier stent, pour le traitement de malformations vasculaires, en particulier d'anévrismes et/ou de fistules, en particulier pour le recouvrement de malformations vasculaires, le dispositif de recouvrement (12) présentant une structure en treillis tubulaire auto-expansible (14) et un couvercle (20) en tissu électrofilé, le couvercle (20) étant relié à la structure en treillis (14) et recouvrant au moins partiellement la structure en treillis (14) pour être placé à l'état implanté sur la malformation vasculaire, le couvercle (20) formant une membrane poreuse, qui est traversable par un moyen d'alimentation (44) pour l'application d'un moyen d'embolisation (40) et est adaptée à se poser sur la circonférence extérieure du moyen d'alimentation (44) une fois qu'il est traversé, la membrane poreuse du couvercle (20) étant conçue pour reformer une ouverture (24) constituée par le moyen d'alimentation lorsqu'il traverse la membrane après le retrait du moyen d'alimentation et représentant au plus 80 %, en particulier au plus 60 %, en particulier au plus 40 %, en particulier au plus 20 % du diamètre du moyen d'alimentation et le couvercle (20) comprenant, sur une surface de 100 000 µm², au moins 10 pores (52) ayant une taille d'au moins 15 µm².
